(19) Europäisches Patentamt / European Patent Office / Office européen des brevets

(11) EP 3 986 366 B1

(12) **EUROPEAN PATENT SPECIFICATION**

(45) Date of publication and mention of the grant of the patent:
**11.09.2024 Bulletin 2024/37**

(21) Application number: **20733816.1**

(22) Date of filing: **23.06.2020**

(51) International Patent Classification (IPC):
***A61K 8/58*** (2006.01) ***A61K 8/892*** (2006.01)
***A61K 8/893*** (2006.01) ***A61Q 5/06*** (2006.01)
***A61Q 5/08*** (2006.01)

(52) Cooperative Patent Classification (CPC):
**A61Q 5/065; A61K 8/58; A61K 8/892; A61K 8/893**

(86) International application number:
**PCT/EP2020/067484**

(87) International publication number:
**WO 2020/260273 (30.12.2020 Gazette 2020/53)**

(54) **COMPOSITION COMPRISING AT LEAST ONE ALKOXYSILANE, AT LEAST ONE NON-AMINO SILICONE AND WATER, THE ALKOXYSILANE/NON-AMINO SILICONE MASS RATIO RANGING FROM 95/5 TO 5/95**

ZUSAMMENSETZUNG MIT MINDESTENS EINEM ALKOXYSILAN, MINDESTENS EINEM NICHT-AMINO-SILIKON UND WASSER, MIT EINEM ALKOXYSILAN / NON-AMINO-SILIKON-MASSENVERHÄLTNIS VON 95/5 BIS 5/95

COMPOSITION COMPRENANT AU MOINS UN ALCOXYSILANE , AU MOINS UNE SILICONE NON AMINÉE ET DE L'EAU, DANS LAQUELLE LE RATIO MASSIQUE D'ALCOXYSILANE/SILICONE NON AMINÉE VARIE DE 95:5 À 5:95.

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.06.2019 FR 1906824**
**24.04.2020 FR 2004110**

(43) Date of publication of application:
**27.04.2022 Bulletin 2022/17**

(73) Proprietor: **L'OREAL**
**75008 Paris (FR)**

(72) Inventors:
- **MARTIN-BESNARDIERE, Julie**
  **93601 AULNAY-SOUS-BOIS (FR)**
- **STEVANT, Boris**
  **93601 AULNAY-SOUS-BOIS (FR)**
- **PROTAT-GERARDIN, Marine**
  **93400 SAINT-OUEN (FR)**
- **LIARD, Alexis**
  **93400 SAINT-OUEN (FR)**

(74) Representative: **Casalonga, Axel**
**Casalonga & Partners**
**Bayerstrasse 71-73**
**80335 München (DE)**

(56) References cited:
**EP-A1- 1 935 398**
**FR-A1- 2 910 297**
**FR-A1- 2 936 414**
**GB-A- 2 186 890**
**US-A1- 2016 166 479**

EP 3 986 366 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention is disclosed in the appended claims.

## Technical field

In the field of dyeing keratin fibres, in particular human keratin fibres, it is already known practice to dye keratin fibres via various techniques using direct dyes for non-permanent dyeing, or dye precursors for permanent dyeing.

Non-permanent dyeing or direct dyeing consists in dyeing keratin fibres with dye compositions containing direct dyes. They are applied to the keratin fibres for a time necessary to obtain the desired colouring, and are then rinsed out.

The standard dyes that are used are, in particular, dyes of the nitrobenzene, anthraquinone, nitropyridine, azo, xanthene, acridine, azine or triarylmethane type, or natural dyes.

Some of these dyes may be used under lightening conditions, which makes it possible to obtain colourings that are visible on dark hair.

It is also known practice to dye keratin fibres permanently via oxidation dyeing. This dyeing technique consists in applying to the keratin fibres a composition containing dye precursors such as oxidation bases and couplers. Under the action of an oxidizing agent, these precursors form one or more coloured substances in the hair.

The variety of molecules used as oxidation bases and couplers makes it possible to obtain a wide range of colours, and the colourings resulting therefrom are generally permanent, strong and resistant to external agents, notably to light, bad weather, washing, perspiration and rubbing.

In order to be visible on dark hair, these two dyeing techniques require prior or simultaneous bleaching of the keratin fibres. This bleaching step, performed with an oxidizing agent such as hydrogen peroxide or persalts, results in appreciable degradation of the keratin fibres, which impairs their cosmetic properties. The hair then has a tendency to become coarse, more difficult to disentangle and more brittle.

Another dyeing method consists in using pigments. Specifically, the use of pigment on the surface of keratin fibres generally makes it possible to obtain colourings that are visible on dark hair, since the surface pigment masks the natural colour of the fibre. However, the colourings obtained via this dyeing method have the drawback of having poor resistance to shampoo washing and also to external agents such as sebum, perspiration, and mechanical actions such as brushing and/or rubbing.

In addition, compositions for temporarily dyeing the hair may also lead to a hair feel that is not natural and/or is uncosmetic, the hair thus dyed possibly notably lacking softness and/or suppleness and/or strand separation.

The need thus remains for a composition for treating keratin fibres, notably the hair, which has the advantage of obtaining a homogeneous and smooth coloured coating on the hair, maintaining complete strand separation of the hair, while at the same time forming a coating that is persistent with respect to shampoo washing and to the various external attacking factors to which the hair may be subjected, such as brushing and/or rubbing, without degrading the hair.

Thus, the aim of the present invention is to develop a composition for treating keratin fibres, notably human keratin fibres such as the hair, which has the advantage of obtaining a homogeneous and smooth coloured coating on the hair, said coating producing complete strand separation of the hair, while at the same time forming a coating that is persistent with respect to shampoo washing and to the various attacking factors to which the hair may be subjected, such as brushing and/or rubbing, without degrading the hair.

FR 2 936 414 A1 (L'OREAL [FR]) seeks to solve the same problem. However, starting from FR 2 936 414 A1 , the skilled person has no incentive to implement a composition according to the invention comprising water as solvent for the purpose of obtaining a hair colouration being resistant to washing out by shampoos.

## Disclosure of the invention

One subject of the present invention is thus a composition (C) for treating keratin fibres, notably human keratin fibres such as the hair, comprising:

a) at least one alkoxysilane chosen from the compounds of formula (I), oligomers thereof and/or mixtures thereof as described below;
b) at least one non-amino silicone chosen from the compounds of formula (III) as described below;
c) water; and
d) at least one colouring agent chosen from pigments, direct dyes and mixtures thereof, said composition being such that the alkoxysilane/non-amino silicone mass ratio ranges from 95/5 to 5/95.

The present invention also relates to a process for treating human keratin fibres such as the hair, which consists in extemporaneously mixing, at the time of use, two compositions (A) and (B) and in applying the mixture to the fibres,

said mixture comprising an alkoxysilane/non-amino silicone mass ratio ranging from 95/5 to 5/95, with:

- composition (A) comprising at least one alkoxysilane chosen from the compounds of formula (I), oligomers thereof and/or mixtures thereof as described below; and water;
- composition (B) comprising at least one non-amino silicone chosen from the compounds of formula (III) as described below; and at least one colouring agent chosen from pigments, direct dyes and mixtures thereof;

it being understood that composition (A) and/or composition (B) comprise water.

The present invention also relates to a device for treating human keratin fibres such as the hair, comprising two compartments containing:

- in a first compartment (C1), a composition (A) comprising at least one alkoxysilane chosen from the compounds of formula (I), oligomers thereof and/or mixtures thereof as described below;
- in a second compartment (C2), a composition (B) comprising at least one non-amino silicone chosen from the compounds of formula (III) as described below; and at least one colouring agent chosen from pigments, direct dyes and mixtures thereof;

it being understood that composition (A) and/or composition (B) comprise water, and the mixing of the compositions of compartments (C1) and (C2) gives an alkoxysilane/non-amino silicone mass ratio ranging from 95/5 to 5/95.

Through the use of this composition (C), coloured coatings are obtained on the hair that make it possible to obtain a colouring that is visible on all types of hair, said colouring being persistent with respect to shampoo washing and preserving the physical qualities of the keratin fibres, without impairing the integrity of the treated fibres. Such a coating may be resistant to the external attacking factors to which the hair may be subjected, such as blow-drying and/or perspiration. It makes it possible in particular to obtain a smooth and uniform deposit.

Moreover, this composition (C) makes it possible to obtain hair with complete strand separation, which can be styled without any problem. The hair after treatment with composition (C) can be subjected to shaping treatments, preferably temporary shaping treatments.

For the purposes of the present invention, the term "*alkoxysilane*" means an alkoxysilane or an alkoxysilane oligomer of formula (I).

The term "*hair with strand separation*" means hair which, after application of the composition and drying, is not stuck together (or of which all the strands are separated from each other) and thus does not form clumps of hair.

For the purposes of the present invention, the term "*colouring that is persistent with respect to shampoo washing*" means that the colouring obtained persists after one shampoo wash, preferably after 3 shampoo washes, more preferentially after 5 shampoo washes, even more preferentially after 10 shampoo washes.

The expression "*including a*" should be understood as meaning "including at least one", unless otherwise specified.

The expression "*at least one*" means one or more.

The invention is not limited to the illustrated examples. The features of the various examples may notably be combined within variants which are not illustrated.

For the purposes of the present invention and unless otherwise indicated:

- the term "*alkyl*" denotes a linear or branched saturated hydrocarbon-based radical containing, for example, from 1 to 20 carbon atoms;

- the term "*aminoalkyl*" denotes an alkyl radical as defined previously, said alkyl radical being substituted with an amino $NH_2$ group;

- the term "*hydroxyalkyl*" denotes an alkyl radical as defined previously, said alkyl radical being substituted with a hydroxyl OH group;

- an "*alkylene*" radical denotes a linear or branched divalent saturated $C_2$-$C_4$ hydrocarbon-based group such as methylene, ethylene or propylene;

- a "*cycloalkyl*" radical denotes a cyclic saturated hydrocarbon-based group comprising from 1 to 3 rings, preferably 2 rings, and comprising from 3 to 20 carbon atoms, preferably between 5 and 10 carbon atoms, such as cyclopentyl, cyclohexyl, cycloheptyl, norbornyl, or isobornyl, the cycloalkyl radical possibly being substituted with one or more ($C_1$-$C_4$)alkyl groups such as methyl; preferably, the cycloalkyl radical is an isobornyl group.

- an "*aryl*" radical is a cyclic unsaturated aromatic radical comprising from 6 to 12 carbon atoms, which is mono- or bicyclic, fused or unfused; preferably, the aryl group consists of 1 ring and contains 6 carbon atoms, such as phenyl;
- the term "*alkoxy*" denotes an alkyl-oxy group with "alkyl" as defined previously.

Composition (C) for treating keratin fibres, notably human keratin fibres such as the hair, according to the invention is preferably a composition for dyeing human keratin fibres such as the hair.

**Alkoxysilane:**

The composition according to the invention comprises at least one alkoxysilane chosen from the compounds of formula (I) below, oligomers thereof and/or mixtures thereof:

[Chem.1] $R^1_xSi(OR^2)_{(4-x)}$ (I)

in which:

- **$R^1$** represents an alkoxy group containing from 1 to 10 carbon atoms, a linear or branched, saturated or unsaturated, cyclic or acyclic $C_1$ to $C_{22}$, notably $C_1$ to $C_{20}$, hydrocarbon-based radical, which may be substituted with at least one group chosen from a hydroxyl group (OH); a thiol group; an amino group $NH_2$; an alkylamino group NHR in which R denotes a linear or branched alkyl radical containing from 1 to 20 carbon atoms, notably from 1 to 10 carbon atoms; an alkoxy group containing from 1 to 10 carbon atoms; a cycloalkyl containing from 3 to 40 carbon atoms; an aryl containing from 6 to 30 carbon atoms; $R^1$ possibly being interrupted with at least one heteroatom chosen from O, S, NH or a carbonyl group (CO);
- **$R^2$** represents a hydrogen atom or an alkyl group containing from 1 to 20 carbon atoms, preferably from 2 to 6 carbon atoms;
- x denotes an integer ranging from 1 to 3.

**$R^1$** and **$R^2$** may be identical or different.

The term "*oligomer*" means compound(s) including at least 2 silicon atoms, obtained by oligomerization or polymerization of the compounds of formula (I).

Preferably, **$R^1$** represents an alkoxy group containing from 1 to 10 carbon atoms, such as an ethoxy, or **$R^1$** represents a linear or branched, saturated $C_1$ to $C_{22}$, notably $C_1$ to $C_{20}$, hydrocarbon-based radical, which is preferably linear, said hydrocarbon-based radical possibly being substituted with at least one amino group $NH_2$ or alkylamino group NHR, in which R denotes a linear or branched $C_1$ to $C_{20}$, notably $C_1$ to $C_{10}$, alkyl.

More preferentially, **$R^1$** represents a saturated linear $C_1$ to $C_6$ hydrocarbon-based radical, which may be substituted with an amino group $NH_2$.

Preferably, **$R^2$** represents an alkyl group containing from 1 to 10 carbon atoms, preferably from 2 to 6 carbon atoms, more preferentially an ethyl.

The alkoxysilane(s) of formula (I), oligomers thereof and/or mixtures thereof may be chosen from:

- the compounds of formula (Ia) and/or (Ib) below, alone or as a mixture:

[Chem.2]

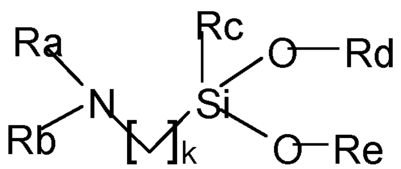

(Ia)

[Chem.3]

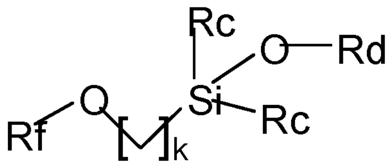

(Ib)

in which:

- **Ra** and **Rb**, which may be identical or different, represent a hydrogen atom; an alkyl group containing from 1 to 20 carbon atoms, preferably from 1 to 10 carbon atoms; a cycloalkyl group containing from 3 to 20 carbon atoms; an aryl group containing from 6 to 12 carbon atoms; an aminoalkyl group containing from 1 to 20 carbon atoms; a hydroxyalkyl group containing from 1 to 20 carbon atoms; an alkoxy group containing from 1 to 10 carbon atoms; an alkylcarbonyl group containing from 1 to 10 carbon atoms, preferably methylcarbonyl (i.e. acetyl);

- **Rc** represents a hydrogen atom; an alkyl group containing from 1 to 20 carbon atoms, preferably a methyl; an alkoxy group containing from 1 to 10 carbon atoms, preferably an ethoxy group; or an alkylaryl group containing from 7 to 12 carbon atoms;

- **Rd** and **Re,** which may be identical or different, represent an alkyl group containing from 1 to 20 carbon atoms, preferably an ethyl;

- **k** denotes an integer ranging from 0 to 20, preferably ranging from 0 to 3;

- **Rf** represents a hydrogen atom; an alkyl group containing from 1 to 20 carbon atoms such as an ethyl group; or a group of formula (II) below:

[Chem.4]

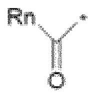

(II)

in which **Rn** represents a hydroxyl group (OH); an alkyl group containing from 1 to 20 carbon atoms, preferably a methyl.

Among the alkoxysilanes of formula (Ia), oligomers thereof and/or mixtures thereof, mention may notably be made of 3-aminopropyltriethoxysilane (APTES), 3-aminopropylmethyldiethoxysilane (APMDES) and N-cyclohexylaminomethyltriethoxysilane.

APTES may be purchased, for example, from the company Dow Corning under the name Xiameter OFS-6011 Silane or from the company Momentive Performance Materials under the name Silsoft A-1100 or from the company Shin-Etsu under the name KBE-903.

The compounds of formula (Ia) may also denote Dynasylan SIVO 210 or Dynasylan 1505 sold by the company Evonik.

N-cyclohexylaminomethyltriethoxysilane may be purchased, for example, from the company Wacker under the name Geniosil XL 926.

Among the alkoxysilanes of formula (Ib), oligomers thereof and/or mixtures thereof, mention may notably be made of tetraethoxysilane (TEOS), methyltriethoxysilane (MTES), dimethyldiethoxysilane (DMDES), diethyldiethoxysilane, dipropyldiethoxysilane, propyltriethoxysilane, isobutyltriethoxysilane, phenyltriethoxysilane, phenylmethyldiethoxysilane, diphenyldiethoxysilane, benzyltriethoxysilane, benzylmethyldiethoxysilane, dibenzyldiethoxysilane, acetoxymethyltriethoxysilane and mixtures thereof.

TEOS may be purchased, for example, from the company Evonik under the name Dynasylan® A or Dynasylan® A SQ. MTES may be purchased, for example, from the company Evonik under the name Dynasylan® MTES. DMDES

may be purchased, for example, from the company Gelest under the reference SID3404.0.

Preferably, the alkoxysilane(s) chosen from the compounds of formula (I), oligomers thereof and/or mixtures thereof are chosen from 3-aminopropyltriethoxysilane (APTES), 3-aminopropylmethyldiethoxysilane (APMDES), N-cyclohexylaminomethyltriethoxysilane, tetraethoxysilane (TEOS), methyltriethoxysilane (MTES), dimethyldiethoxysilane (DMDES), diethyldiethoxysilane, dipropyldiethoxysilane, propyltriethoxysilane, isobutyltriethoxysilane, phenyltriethoxysilane, phenylmethyldiethoxysilane, diphenyldiethoxysilane, benzyltriethoxysilane, benzylmethyldiethoxysilane, dibenzyldiethoxysilane, acetoxymethyltriethoxysilane and mixtures thereof, more preferentially 3-aminopropyltriethoxysilane (APTES), tetraethoxysilane (TEOS) and mixtures thereof.

According to a preferred embodiment, the alkoxysilane(s) of formula (I), oligomers thereof and/or mixtures thereof are chosen from the compounds of formula (Ia) below:

[Chem.5]

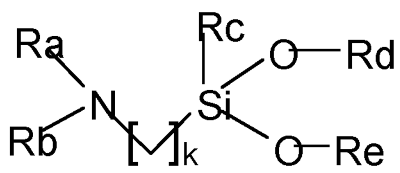

(Ia)

in which:

- **Ra** and **Rb**, which are identical, represent a hydrogen atom;
- **Rc** represents an alkyl group containing from 1 to 20 carbon atoms, preferably a methyl, or an alkoxy group containing from 1 to 10 carbon atoms, preferably an ethoxy group;
- **Rd** and **Re,** which may be identical or different, represent an alkyl group containing from 1 to 20 carbon atoms, preferably an ethyl;
- **k** denotes an integer ranging from 0 to 20, preferably ranging from 0 to 3.

Preferentially, **Ra** and **Rb** represent a hydrogen atom, **Rc** represents an ethoxy group, **Rd** and **Re** are identical and represent an ethyl and k is equal to 3.

According to a more preferred embodiment, the alkoxysilane of formula (I), oligomers thereof and/or mixtures thereof, is 3-aminopropyltriethoxysilane (APTES).

According to another preferred embodiment, the alkoxysilane(s) of formula (I), oligomers thereof and/or mixtures thereof are chosen from the compounds of formula (Ib) below:

[Chem.6]

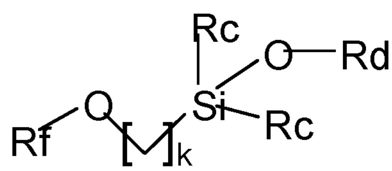

(Ib)

in which

- **Rc** represents an alkoxy group containing from 1 to 10 carbon atoms, preferably an ethoxy group;
- **Rd** represents an alkyl group containing from 1 to 20 carbon atoms, preferably an ethyl;

- **k** denotes an integer ranging from 0 to 20, preferably ranging from 0 to 3;
- **Rf** represents a hydrogen atom or an alkyl group containing from 1 to 20 carbon atoms, such as an ethyl;

Composition (C) according to the invention may comprise one or more alkoxysilanes of formula (I), oligomers thereof and/or mixtures thereof present in a total amount ranging from 0.1% to 30% by weight, preferably from 0.5% to 25% by weight, better still from 0.5% to 20% by weight, relative to the total weight of the composition.

**Non-amino silicone:**

The composition according to the invention comprises at least one non-amino silicone chosen from the compounds of formula (III) below:

[Chem. 7]

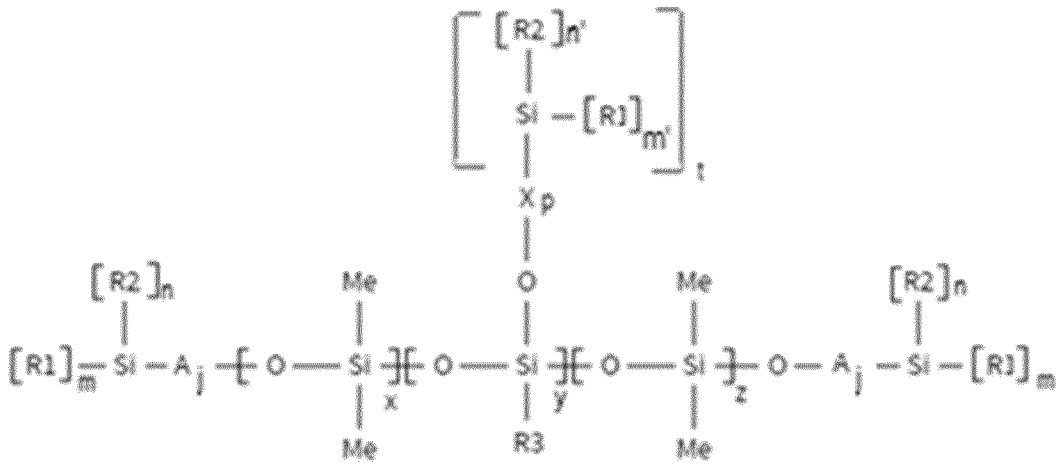

(III)

in which:

- **R1** independently represents a hydroxyl group or an alkoxy group containing from 1 to 2 carbon atoms;
- **R2** independently represents a hydrogen atom; an alkyl group containing from 1 to 20 carbon atoms, optionally substituted with at least one group chosen from a hydroxyl group (OH) or a thiol group, said alkyl group preferably being optionally substituted with at least one hydroxyl group (OH);
- **R3** represents a hydrogen atom; a hydroxyl group; an alkyl group containing from 1 to 10 carbon atoms, optionally substituted with at least one group chosen from a hydroxyl group (OH) or a thiol group; a cycloalkyl group containing from 3 to 20 carbon atoms, optionally substituted with at least one group chosen from a hydroxyl group (OH) or a thiol group; an alkoxy group containing from 1 to 2 carbon atoms, optionally substituted with at least one group chosen from a hydroxyl group (OH) or a thiol group; an aryl group containing from 6 to 12 carbon atoms, optionally substituted with at least one group chosen from a hydroxyl group (OH) or a thiol group;
- **A** represents an alkylene group containing from 1 to 2 carbon atoms;
- **X** represents a hydrogen atom, an alkyl group containing from 1 to 2 carbon atoms, a group $-(R4-O)_p-$ with **R4** representing a linear or branched alkylene group containing from 2 to 4 carbon atoms and **p** denoting an integer ranging from 0 to 3;
- **m** denotes an integer ranging from 1 to 3, **n** denotes an integer ranging from 0 to 2 and m+n=3;
- **m'** denotes an integer ranging from 1 to 3, **n'** denotes an integer ranging from 0 to 2 and m'+n'=3;

- **j** denotes an integer ranging from 0 to 1;
- **t** denotes an integer ranging from 0 to 1;
- **y** denotes an integer ranging from 0 to 10, z denotes an integer ranging from 0 to 500, with x+z ranging from 0 to 500 and x+y+z≥4; and

it being understood that if X represents a hydrogen atom, then t=0 and if p=0, then t=1.

The term "*non-amino silicone*" means any silicone not comprising any primary, secondary or tertiary amine groups or quaternary ammonium groups.

According to a preferred embodiment of the present invention, the compound(s) of formula (III) are such that:

- **R1** independently represents a hydroxyl group or an alkoxy group containing from 1 to 2 carbon atoms;
- **R2** independently represents a hydrogen atom or an alkyl group containing from 1 to 10 carbon atoms, preferably a methyl;
- **R3** represents a hydrogen atom; a hydroxyl group; an alkyl group containing from 1 to 10 carbon atoms; an alkoxy group containing from 1 to 2 carbon atoms or an aryl group containing from 6 to 12 carbon atoms, optionally substituted with a group chosen from a hydroxyl group (OH) or a thiol group;
- **X** represents a hydrogen atom or an alkyl group containing from 1 to 2 carbon atoms, and **p** denotes an integer ranging from 0 to 3;
- **m'** denotes an integer ranging from 1 to 3, **n'** denotes an integer ranging from 0 to 2 and m'+n'=3;
- **m** denotes an integer ranging from 1 to 3, **n** denotes an integer ranging from 0 to 2 and m+n=3;
- **j** is equal to 0;
- **t** denotes an integer ranging from 0 to 1; and
- **y** denotes an integer ranging from 0 to 10, z denotes an integer ranging from 0 to 500, with x+z ranging from 0 to 500 and x+y+z≥4; and

it being understood that if X represents a hydrogen atom, then t=0 and if p=0, then t=1.

According to an even more preferred embodiment of the present invention, the compound(s) of formula (III) are such that:

- **R1** independently represents a hydroxyl group or an alkoxy group containing from 1 to 2 carbon atoms;
- **R2** independently represents an alkyl group containing from 1 to 10 carbon atoms, preferably a methyl;
- **m** denotes an integer ranging from 1 to 3, n denotes an integer ranging from 0 to 2 and m+n=3;
- **j** is equal to 0;
- **y** is equal to 0;
- **z** denotes an integer ranging from 0 to 500; and
- $$x+z \geq 4.$$

The non-amino silicone(s) of formula (III) that may be used in the context of the invention may be chosen from:

- the compounds of formula (IIIa) below:

[Chem.8]

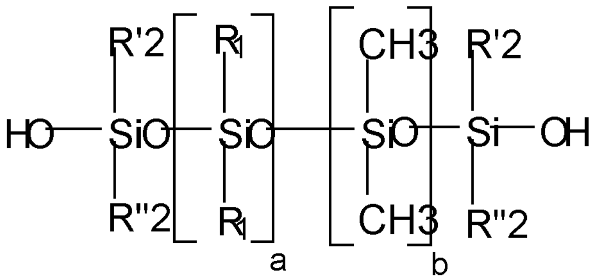

(IIIa)

in which:

- $R_1$ independently represents a hydroxyl group; an alkyl group containing from 1 to 10 carbon atoms, preferably a methyl; an aryl group containing from 6 to 12 carbon atoms; an alkoxy group containing from 1 to 2 carbon atoms; or a group -O-Si(R'$_2$)$_3$;
- $R'_2$ and $R''_2$ independently represent a hydrogen atom; a hydroxyl group; or an alkyl group containing from 1 to 10 carbon atoms, preferably a methyl;
- **a** denotes an integer ranging from 0 to 10, **b** denotes an integer ranging from 0 to 500, with a+b≥4.

- the compounds of formula (IIIb) below:

[Chem. 9]

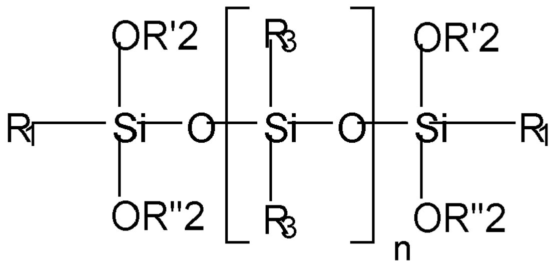

(IIIb)

in which:

- $R_1$ represents an alkyl group containing from 1 to 10 carbon atoms, preferably a methyl, or an alkoxy group containing from 1 to 2 carbon atoms;
- $R'_2$ and $R''_2$ independently represent an alkyl group containing from 1 to 10 carbon atoms, preferably containing 1 or 2 carbon atoms;
- $R_3$ represents an alkyl group containing from 1 to 10 carbon atoms, preferably a methyl; an alkoxy group containing from 1 to 2 carbon atoms; or a group -O-Si($R_1$)$_3$; and
- **n** denotes an integer ranging from 0 to 10.

- the compounds of formula (IIIc) below:

[Chem. 10]

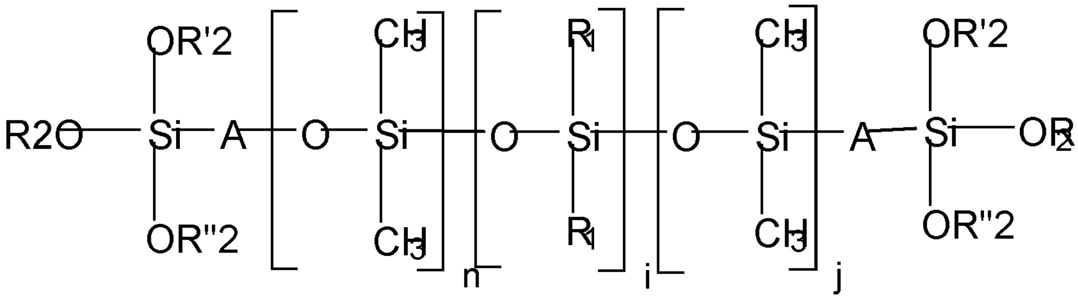

(IIIc)

in which:

- $\mathbf{R_1}$ represents an alkyl group containing from 1 to 10 carbon atoms, preferably a methyl; an alkoxy group containing from 1 to 2 carbon atoms; or a group $-O-X-Si(OR_2)_3$ with **X** representing an alkylene group containing from 1 to 2 carbon atoms, a group $-(O-R_4)_p-$ with $\mathbf{R_4}$ representing a linear or branched alkylene group containing from 2 to 4 carbon atoms and **p** denoting an integer ranging from 0 to 3;
- $\mathbf{R_2}$, $\mathbf{R'_2}$ and $\mathbf{R''_2}$ independently represent an alkyl group containing from 1 to 10 carbon atoms, preferably a methyl or ethyl;
- **A** represents an alkylene group containing from 1 to 2 carbon atoms;
- **j** denotes an integer ranging from 0 to 1;
- i denotes an integer ranging from 0 to 10, **n+i** ranging from 0 to 500, with $n+i+j \geq 4$;

- the compounds of formula (IIId) below:

[Chem. 11]

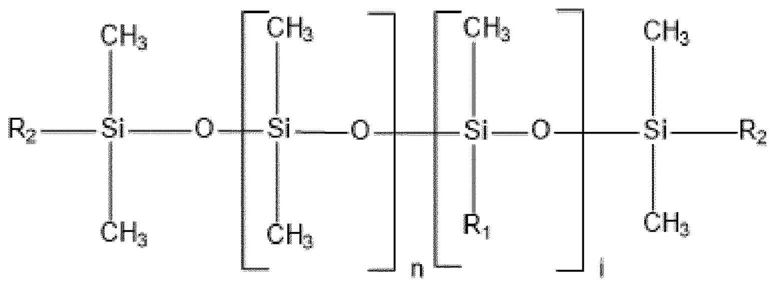

(IIId)

in which:

- $\mathbf{R_1}$ independently represents an alkyl group containing from 1 to 10 carbon atoms, preferably from 1 to 4 carbon atoms, more preferentially a methyl; or an alkoxy group containing from 1 to 2 carbon atoms;
- $\mathbf{R_2}$, which may be identical or different, independently represent a hydroxyl group or an alkoxy group containing from 1 to 2 carbon atoms;
- **i** denotes an integer ranging from 0 to 18, **n** denotes an integer ranging from 0 to 18 with **n+i** ranging from 4 to 18.

- and mixtures thereof.

Preferably, the non-amino silicone(s) of formula (IIId) are such that:

- **$R_1$** independently represents an alkyl group containing from 1 to 10 carbon atoms, preferably from 1 to 4 carbon atoms, more preferentially a methyl;
- **$R_2$** independently represents a hydroxyl group or an alkoxy group containing from 1 to 2 carbon atoms;
- **i** denotes an integer ranging from 0 to 18, **n** denotes an integer ranging from 0 to 18 with **n+i** ranging from 4 to 18.

More preferentially, the non-amino silicone(s) of formula (III d) are such that:

- **$R_1$** independently represents an alkyl group containing from 1 to 4 carbon atoms, more preferentially a methyl;
- **$R_2$** independently represents a hydroxyl group;
- **i** denotes an integer ranging from 0 to 18, **n** denotes an integer ranging from 0 to 18 with **n+i** ranging from 4 to 18.

The term "*non-amino silicone*" denotes any silicone not comprising any primary, secondary or tertiary amine groups or quaternary ammonium groups.

Among the non-amino silicones of formula (IIIa), mention may be made of polydimethylsiloxanes (PDMS) bearing hydroxyl end functions, such as the compounds sold by the company Shin-Etsu under the name KF-9701 or X-21-5841, or those sold by the company Sigma-Aldrich under the reference 481939 (Mn ~550, ~25 cSt), 481955 (~65 cSt), or 481963 (~750 cSt).

Among the non-amino silicones of formula (IIIb), mention may be made of polydimethylsiloxanes (PDMS) bearing tri(methoxy) end functions, such as those sold by the company Power Chemical under the name SiSiB® PF2110, or those sold by the company Shin-Etsu under the name X40-9246, KC-89S or X40-9250. Mention may also be made of polydimethylsiloxanes (PDMS) bearing tri(ethoxy) end functions, such as those sold by the company Power Chemical under the name SiSiB® PF2120.

Among the non-amino silicones of formula (IIIc), mention may be made of polydimethylsiloxanes (PDMS) bearing tri(ethoxy) end functions, such as those sold by the company Gelest under the name DMS-XT11.

Among the non-amino silicones of formula (IIId), mention may be made of polydimethylsiloxanes (PDMS) bearing alkoxy end functions, such as those sold by the company Gelest under the reference DMS-XM11 or polydimethylsiloxanes (PDMS) bearing hydroxyl end functions, such as those sold by the company Sigma-Aldrich under the reference 481939 (Mn ~550).

Preferably, the non-amino silicone(s) that may be used in the context of the invention are chosen from the compounds of formula (IIIa) in which:

- **$R_1$** independently represents an alkyl group containing from 1 to 10 carbon atoms, preferably a methyl;
- **$R'_2$** and **$R''_2$** independently represent a hydroxyl group or an alkyl group containing from 1 to 10 carbon atoms, preferably an alkyl group, more preferentially a methyl;
- **b** denotes an integer ranging from 0 to 10, **a** denotes an integer ranging from 0 to 5, with $a+b \geq 4$.

Preferably, the non-amino silicone(s) of formula (III) that may be used in the context of the invention are represented by formula (IV) below:

[Chem. 12]

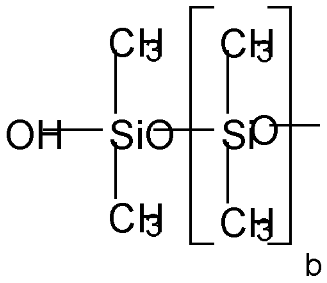

(IV)

in which b denotes an integer ranging from 0 to 20, preferably from 4 to 18, more preferably from 4 to 10.

Preferably, the non-amino silicone(s) that may be used in the context of the invention have a molecular mass of between 400 and 1800 g/mol.

The non-amino silicone(s) of formula (IIId) have a weight-average molecular mass of less than or equal to 1500 g/mol.

Preferably, the non-amino silicone(s) of formula (IIId) have a weight-average molecular mass of less than or equal to 1300 g/mol.

Preferably, the non-amino silicone(s) of formula (IIId) have a weight-average molecular mass ranging from 460 to 1500 g/mol, more preferentially from 500 to 1300 g/mol.

Composition (C) according to the invention may comprise one or more non-amino silicones of formula (III) present in a total amount ranging from 0.1% to 30% by weight, preferably from 0.1% to 25% by weight, relative to the total weight of the composition.

**Mass ratio:**

Composition (C) according to the invention comprises an alkoxysilane/non-amino silicone mass ratio ranging from 95/5 to 5/95.

Preferably, the alkoxysilane/non-amino silicone mass ratio ranges from 90/10 to 10/90, preferentially from 60/40 to 40/60; even more preferentially, the alkoxysilane/non-amino silicone mass ratio is 50/50.

According to a particular embodiment, the APTES/non-amino silicone mass ratio ranges from 90/10 to 10/90, preferably 50/50.

According to a particular embodiment, the TEOS/non-amino silicone mass ratio ranges from 90/10 to 10/90, preferably 50/50.

According to a particular embodiment, the TEOS and APTES/non-amino silicone mass ratio is 50/50 with 25% of TEOS and 25% of APTES.

According to a particular embodiment, the APTES/non-amino silicone(s) of formula (IIIa) or (IIId) mass ratio ranges from 90/10 to 10/90, preferentially from 80/20 to 20/80, and more preferentially from 70/30 to 30/70.

According to a particular embodiment, the APTES/non-amino silicone(s) of formula (IV) mass ratio ranges from 90/10 to 10/90, more preferentially from 70/30 to 30/70.

**Dye:**

Composition (C) according to the invention comprises at least one colouring agent chosen from pigments, direct dyes and mixtures thereof.

Preferably, composition (C) according to the invention comprises one or more pigments.

The term "pigment" means any pigment that gives colour to keratin materials. Their solubility in water at 25°C and at atmospheric pressure (760 mmHg) is less than 0.05% by weight, and preferably less than 0.01%.

The pigments that may be used are notably chosen from the organic and/or mineral pigments known in the art, notably those that are described in Kirk-Othmer's Encyclopedia of Chemical Technology and in Ullmann's Encyclopedia of Industrial Chemistry.

They may be natural, of natural origin, or non-natural.

These pigments may be in pigment powder or paste form. They may be coated or uncoated.

The pigments may be chosen, for example, from mineral pigments, organic pigments, lakes, pigments with special effects such as nacres or glitter flakes, and mixtures thereof.

The pigment may be a mineral pigment. The term "mineral pigment" means any pigment that satisfies the

definition in Ullmann's encyclopedia in the chapter on inorganic pigments. Among the mineral pigments that are useful in the present invention, mention may be made of iron oxides, chromium oxides, manganese violet, ultramarine blue, chromium hydrate, ferric blue and titanium oxide.

The pigment may be an organic pigment. The term "organic pigment" means any pigment that satisfies the definition in Ullmann's encyclopedia in the chapter on organic pigments.

The organic pigment may notably be chosen from nitroso, nitro, azo, xanthene, pyrene, quinoline, anthraquinone, triphenylmethane, fluorane, phthalocyanine, metal-complex, isoindolinone, isoindoline, quinacridone, perinone, perylene, diketopyrrolopyrrole, indigo, thioindigo, dioxazine, triphenylmethane and quinophthalone compounds.

In particular, the white or coloured organic pigments may be chosen from carmine, carbon black, aniline black, azo yellow, quinacridone, phthalocyanine blue, the blue pigments codified in the Color Index under the references CI 42090, 69800, 69825, 74100, 74160, the yellow pigments codified in the Color Index under the references CI 11680, 11710, 19140, 20040, 21100, 21108, 47000, 47005, the green pigments codified in the Color Index under the references CI 61565, 61570, 74260, the orange pigments codified in the Color Index under the references CI 11725, 45370, 71105, the red pigments codified in the Color Index under the references CI 12085, 12120, 12370, 12420, 12490, 14700, 15525, 15580, 15620, 15630, 15800, 15850, 15865, 15880, 26100, 45380, 45410, 58000, 73360, 73915, 75470, the pigments obtained by oxidative polymerization of indole or phenol derivatives as described in patent FR 2 679 771.

Examples that may also be mentioned include pigment pastes of organic pigments, such as the products sold by the company Hoechst under the names:

- Cosmenyl Yellow IOG: Yellow 3 pigment (CI 11710);
- Cosmenyl Yellow G: Yellow 1 pigment (CI 11680);
- Cosmenyl Orange GR: Orange 43 pigment (CI 71105);
- Cosmenyl Red R: Red 4 pigment (CI 12085);
- Cosmenyl Carmine FB: Red 5 pigment (CI 12490);
- Cosmenyl Violet RL: Violet 23 pigment (CI 51319);
- Cosmenyl Blue A2R: Blue 15.1 pigment (CI 74160);
- Cosmenyl Green GG: Green 7 pigment (CI 74260);
- Cosmenyl Black R: Black 7 pigment (CI 77266).

The pigments in accordance with the invention may also be in the form of composite pigments, as described in patent EP 1 184 426. These composite pigments may be composed notably of particles including a mineral core, at least one binder, for attaching the organic pigments to the core, and at least one organic pigment which at least partially covers the core.

The organic pigment may also be a lake. The term "lake" means dyes adsorbed onto insoluble particles, the assembly thus obtained remaining insoluble during use.

The inorganic substrates onto which the dyes are adsorbed are, for example, alumina, silica, calcium sodium borosilicate, calcium aluminium borosilicate and aluminium.

Among the dyes, mention may be made of carminic acid. Mention may also be made of the dyes known under the following names: D&C Red 21 (CI 45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), D&C Yellow 5 (CI 19 140), D&C Yellow 6 (CI 15 985), D&C Green (CI 61 570), D&C Yellow 10 (CI 77 002), D&C Green 3 (CI 42 053), D&C Blue 1 (CI 42 090).

An example of a lake that may be mentioned is the product known under the following name: D&C Red 7 (CI 15 850:1).

The pigment may also be a pigment with special effects. The term "pigments with special effects" means pigments that generally create a coloured appearance (characterized by a certain shade, a certain vivacity and a certain level of luminance) that is non-uniform and that changes as a function of the conditions of observation (light, temperature, angles of observation, etc.). They thereby differ from coloured pigments, which afford a standard uniform opaque, semi-transparent or transparent shade.

Several types of pigments with special effects exist: those with a low refractive index, such as fluorescent or

photochromic pigments, and those with a higher refractive index, such as nacres, interference pigments or glitter flakes.

Examples of pigments with special effects that may be mentioned include nacreous pigments such as mica coated with titanium or with bismuth oxychloride, coloured nacreous pigments such as mica coated with titanium and with iron oxides, mica coated with iron oxide, mica coated with titanium and notably with ferric blue or with chromium oxide, mica coated with titanium and with an organic pigment as defined above, and also nacreous pigments based on bismuth oxychloride. Nacreous pigments that may be mentioned include the Cellini nacres sold by BASF (mica-TiO2-lake), Prestige sold by Eckart (mica-TiO2), Prestige Bronze sold by Eckart (mica-Fe2O3), and Colorona sold by Merck (mica-TiO2-Fe2O3).

Mention may also be made of the gold-coloured nacres sold notably by the company BASF under the name Brilliant gold 212G (Timica), Gold 222C (Cloisonne), Sparkle gold (Timica), Gold 4504 (Chromalite) and Monarch gold 233X (Cloisonne); the bronze nacres sold notably by the company Merck under the name Bronze fine (17384) (Colorona) and Bronze (17353) (Colorona) and by the company BASF under the name Super bronze (Cloisonne); the orange nacres sold notably by the company BASF under the name Orange 363C (Cloisonne) and Orange MCR 101 (Cosmica) and by the company Merck under the name Passion orange (Colorona) and Matte orange (17449) (Microna); the brown nacres sold notably by the company BASF under the name Nu-antique copper 340XB (Cloisonne) and Brown CL4509 (Chromalite); the nacres with a copper tint sold notably by the company BASF under the name Copper 340A (Timica); the nacres with a red tint sold notably by the company Merck under the name Sienna fine (17386) (Colorona); the nacres with a yellow tint sold notably by the company BASF under the name Yellow (4502) (Chromalite); the red nacres with a gold tint sold notably by the company BASF under the name Sunstone G012 (Gemtone); the pink nacres sold notably by the company BASF under the name Tan opale G005 (Gemtone); the black nacres with a gold tint sold notably by the company BASF under the name Nu antique bronze 240 AB (Timica), the blue nacres sold notably by the company Merck under the name Matte blue (17433) (Microna), the white nacres with a silvery tint sold notably by the company Merck under the name Xirona Silver, and the golden-green pink-orange nacres sold notably by the company Merck under the name Indian summer (Xirona), and mixtures thereof.

Still as examples of nacres, mention may also be made of particles including a borosilicate substrate coated with titanium oxide.

Particles comprising a glass substrate coated with titanium oxide are notably sold under the name Metashine MC1080RY by the company Toyal.

Finally, examples of nacres that may also be mentioned include polyethylene terephthalate flakes, notably those sold by the company Meadowbrook Inventions under the name Silver 1P 0.004X0.004 (silver flakes). Multilayer pigments based on synthetic substrates such as alumina, silica, calcium sodium borosilicate or calcium aluminium borosilicate, and aluminium, may also be envisaged.

The pigments with special effects may also be chosen from reflective particles, i.e. notably from particles whose size, structure, notably the thickness of the layer(s) of which they are made and their physical and chemical nature, and surface state, allow them to reflect incident light. This reflection may, where appropriate, have an intensity sufficient to create at the surface of the composition or of the mixture, when it is applied to the support to be made up, highlight points that are visible to the naked eye, i.e. more luminous points that contrast with their environment by appearing to sparkle.

The reflective particles may be selected so as not to significantly alter the colouring effect generated by the colouring agents with which they are combined, and more particularly so as to optimize this effect in terms of colour rendition. They may more particularly have a yellow, pink, red, bronze, orange, brown, gold and/or coppery colour or tint.

These particles may have varied forms and may notably be in platelet or globular form, in particular in spherical form.

Irrespective of their form, the reflective particles may or may not have a multilayer structure, and, in the case of a multilayer structure, may have, for example, at least one layer of uniform thickness, notably of a reflective material.

When the reflective particles do not have a multilayer structure, they may be composed, for example, of metal oxides, notably titanium or iron oxides obtained synthetically.

When the reflective particles have a multilayer structure, they may include, for example, a natural or synthetic substrate, notably a synthetic substrate at least partially coated with at least one layer of a reflective material, notably of at least one metal or metallic material. The substrate may be made of one or more organic and/or mineral materials.

More particularly, it may be chosen from glasses, ceramics, graphite, metal oxides, aluminas, silicas, silicates, notably aluminosilicates and borosilicates, synthetic mica and mixtures thereof, this list not being limiting.

The reflective material may include a layer of metal or of a metallic material.

Reflective particles are notably described in JP-A-09188830, JP-A-10158450, JP-A-10158541, JP-A-07258460 and JP-A-05017710.

Still as an example of reflective particles including a mineral substrate coated with a layer of metal, mention may also be made of particles including a silver-coated borosilicate substrate.

Particles with a silver-coated glass substrate, in the form of platelets, are sold under the name Microglass

Metashine REFSX 2025 PS by the company Toyal. Particles with a glass substrate coated with nickel/chromium/molybdenum alloy are sold under the names Crystal Star GF 550 and GF 2525 by this same company.

Use may also be made of particles comprising a metal substrate, such as silver, aluminium, iron, chromium, nickel, molybdenum, gold, copper, zinc, tin, magnesium, steel, bronze or titanium, said substrate being coated with at least one layer of at least one metal oxide, such as titanium oxide, aluminium oxide, iron oxide, cerium oxide, chromium oxide, silicon oxides and mixtures thereof.

Examples that may be mentioned include aluminium powder, bronze powder or copper powder coated with $SiO_2$ sold under the name Visionaire by the company Eckart.

Mention may also be made of pigments with an interference effect which are not attached to a substrate, such as liquid crystals (Helicones HC from Wacker) or interference holographic glitter flakes (Geometric Pigments or Spectra f/x from Spectratek). Pigments with special effects also comprise fluorescent pigments, whether these are substances that are fluorescent in daylight or that produce an ultraviolet fluorescence, phosphorescent pigments, photochromic pigments, thermochromic pigments and quantum dots, sold, for example, by the company Quantum Dots Corporation.

The variety of pigments that may be used in the present invention makes it possible to obtain a wide range of colours, and also particular optical effects such as metallic effects or interference effects.

The size of the pigment used in the composition according to the present invention is generally between 10 nm and 200 μm, preferably between 20 nm and 80 μm and more preferentially between 30 nm and 50 μm.

The pigments may be dispersed in the composition by means of a dispersant.

The dispersant serves to protect the dispersed particles against their agglomeration or flocculation. This dispersant may be a surfactant, an oligomer, a polymer or a mixture of several thereof, bearing one or more functionalities with a strong affinity for the surface of the particles to be dispersed. In particular, they may become physically or chemically attached to the surface of the pigments. These dispersants also contain at least one functional group that is compatible with or soluble in the continuous medium. In particular, use is made of esters of 12-hydroxystearic acid, in particular, and of $C_8$ to $C_{20}$ fatty acids and of polyols such as glycerol or diglycerol, such as poly(12-hydroxystearic acid) stearate with a molecular weight of approximately 750g/mol, such as the product sold under the name Solsperse 21 000 by the company Avecia, polyglyceryl-2 dipolyhydroxystearate (CTFA name) sold under the reference Dehymyls PGPH by the company Henkel, or polyhydroxystearic acid such as the product sold under the reference Arlacel P100 by the company Uniqema, and mixtures thereof.

As other dispersants that may be used in the compositions of the invention, mention may be made of quaternary ammonium derivatives of polycondensed fatty acids, for instance Solsperse 17 000 sold by the company Avecia, and polydimethylsiloxane/oxypropylene mixtures such as those sold by the company Dow Coming under the references DC2-5185 and DC2-5225 C.

The pigments used in the composition may be surface-treated with an organic agent.

Thus, the pigments that have been surface-treated beforehand, which are useful in the context of the invention, are pigments that have totally or partially undergone a surface treatment of chemical, electronic, electrochemical, mechanochemical or mechanical nature, with an organic agent such as those that are described notably in Cosmetics and Toiletries, February 1990, Vol. 105, pages 53-64, before being dispersed in the composition in accordance with the invention. These organic agents may be chosen, for example, from waxes, for example carnauba wax and beeswax; fatty acids, fatty alcohols and derivatives thereof, such as stearic acid, hydroxystearic acid, stearyl alcohol, hydroxystearyl alcohol, lauric acid and derivatives thereof; anionic surfactants; lecithins; sodium, potassium, magnesium, iron, titanium, zinc or aluminium salts of fatty acids, for example aluminium stearate or laurate; metal alkoxides; polyethylene; (meth)acrylic polymers, for example polymethyl methacrylates; polymers and copolymers containing acrylate units; alkanolamines; silicone compounds, for example silicones, notably polydimethylsiloxanes; organofluorine compounds, for example perfluoroalkyl ethers; fluorosilicone compounds.

The surface-treated pigments that are useful in the composition may also have been treated with a mixture of these compounds and/or may have undergone several surface treatments.

The surface-treated pigments that are useful in the context of the present invention may be prepared according to surface-treatment techniques that are well known to those skilled in the art, or may be commercially available as is.

Preferably, the surface-treated pigments are coated with an organic layer.

The organic agent with which the pigments are treated may be deposited on the pigments by solvent evaporation, chemical reaction between the molecules of the surface agent or creation of a covalent bond between the surface agent and the pigments.

The surface treatment may thus be performed, for example, by chemical reaction of a surface agent with the surface of the pigments and creation of a covalent bond between the surface agent and the pigments or the fillers. This method is notably described in patent US 4 578 266.

An organic agent covalently bonded to the pigments will preferably be used.

The agent for the surface treatment may represent from 0.1% to 50% by weight of the total weight of the surface-treated pigment, preferably from 0.5% to 30% by weight and even more preferentially from 1% to 20% by weight of the

total weight of the surface-treated pigment.

Preferably, the surface treatments of the pigments are chosen from the following treatments:

- a PEG-silicone treatment, for instance the AQ surface treatment sold by LCW;
- a methicone treatment, for instance the SI surface treatment sold by LCW;
- a dimethicone treatment, for instance the Covasil 3.05 surface treatment sold by LCW;
- a dimethicone/trimethyl siloxysilicate treatment, for instance the Covasil 4.05 surface treatment sold by LCW;
- a magnesium myristate treatment, for instance the MM surface treatment sold by LCW;
- an aluminium dimyristate treatment, such as the MI surface treatment sold by Miyoshi;
- a perfluoropolymethylisopropyl ether treatment, for instance the FHC surface treatment sold by LCW;
- an isostearyl sebacate treatment, for instance the HS surface treatment sold by Miyoshi;
- a perfluoroalkyl phosphate treatment, for instance the PF surface treatment sold by Daito;
- an acrylate/dimethicone copolymer and perfluoroalkyl phosphate treatment, for instance the FSA surface treatment sold by Daito;
- a polymethylhydrosiloxane/perfluoroalkyl phosphate treatment, for instance the FS01 surface treatment sold by Daito;
- an acrylate/dimethicone copolymer treatment, for instance the ASC surface treatment sold by Daito;
- an isopropyl titanium triisostearate treatment, for instance the ITT surface treatment sold by Daito;
- an acrylate copolymer treatment, for instance the APD surface treatment sold by Daito;
- a perfluoroalkyl phosphate/isopropyl titanium triisostearate treatment, for instance the PF + ITT surface treatment sold by Daito.

According to a particular embodiment of the invention, the dispersant is present with organic or mineral pigments in submicron-sized particulate form in the dye composition.

The term "submicron" or "submicronic" refers to pigments having a particle size that has been micronized by a micronization method and having a mean particle size of less than a micrometre ($\mu$m), in particular between 0.1 and 0.9 $\mu$m, and preferably between 0.2 and 0.6 $\mu$m.

According to one embodiment, the dispersant and the pigment(s) are present in an amount (dispersant:pigment) of between 1:4 and 4:1, particularly between 1.5:3.5 and 3.5:1 or better still between 1.75:3 and 3:1.

The dispersant(s) may therefore have a silicone backbone, such as silicone polyether and dispersants of aminosilicone type other than the alkoxysilanes described previously. Among the suitable dispersants, mention may be made of:

- aminosilicones, i.e. silicones comprising one or more amino groups such as those sold under the names and references: BYK LPX 21879 by BYK, GP-4, GP-6, GP-344, GP-851, GP-965, GP-967 and GP-988-1, sold by Genesee Polymers,
- silicone acrylates such as Tego® RC 902, Tego® RC 922, Tego® RC 1041, and Tego® RC 1043, sold by Evonik,
- polydimethylsiloxane (PDMS) silicones with carboxyl groups such as X- 22162 and X-22370 by Shin-Etsu, epoxy silicones such as GP-29, GP-32, GP-502, GP-504, GP-514, GP-607, GP-682, and GP-695 by Genesee Polymers, or Tego® RC 1401, Tego® RC 1403, Tego® RC 1412 by Evonik.

According to a particular embodiment, the dispersant(s) are of aminosilicone type other than the alkoxysilanes

described previously and are cationic.

Preferably, the pigment(s) is (are) chosen from mineral, mixed mineral-organic or organic pigments.

In one variant of the invention, the pigment(s) according to the invention are organic pigments, preferentially organic pigments surface-treated with an organic agent chosen from silicone compounds. In another variant of the invention, the pigment(s) according to the invention are mineral pigments.

The composition may comprise one or more direct dyes.

The term "direct dye" means natural and/or synthetic dyes, other than oxidation dyes. These are dyes that will spread superficially on the fibre.

They may be ionic or nonionic, preferably cationic or nonionic.

Examples of suitable direct dyes that may be mentioned include azo direct dyes; (poly)methine dyes such as cyanines, hemicyanines and styryls; carbonyl dyes; azine dyes; nitro(hetero)aryl dyes; tri(hetero)arylmethane dyes; porphyrin dyes; phthalocyanine dyes and natural direct dyes, alone or in the form of mixtures.

The direct dyes are preferably cationic direct dyes. Mention may be made of the hydrazono cationic dyes of formulae (V) and (VI) and the azo cationic dyes (VII) and (VIII) below:

[Chem. 13] $Het^+$-C(Ra)=N-N(Rb)-Ar, Q (V)

[Chem. 14] $Het^+$-N(Ra)-N=C(Rb)-Ar, Q- (VI)

[Chem. 15] $Het^+$-N=N-Ar, Q- (VII)

[Chem. 16] $Ar^+$-N=N-Ar", Q- (VIII)

in which formulae (V) to (VIII):

- **$Het^+$** represents a cationic heteroaryl radical, preferentially bearing an endocyclic cationic charge, such as imidazolium, indolium or pyridinium, which is optionally substituted, preferentially with at least one ($C_1$-$C_8$) alkyl group such as methyl;

- **$Ar^+$** represents an aryl radical, such as phenyl or naphthyl, bearing an exocyclic cationic charge, preferentially ammonium, particularly tri($C_1$-$C_8$)alkylammonium, such as trimethylammonium;

- **Ar** represents an aryl group, notably phenyl, which is optionally substituted, preferentially with one or more electron-donating groups such as i) optionally substituted ($C_1$-$C_8$)alkyl, ii) optionally substituted (Ci-Cs)alkoxy, iii) (di)($C_1$-$C_8$)(alkyl)amino optionally substituted on the alkyl group(s) with a hydroxyl group, iv) aryl($C_1$-$C_8$)alkylamino, v) optionally substituted N-($C_1$-$C_8$)alkyl-N-aryl($C_1$-$C_8$)alkylamino or alternatively Ar represents a julolidine group;

- **Ar"** represents an optionally substituted (hetero)aryl group, such as phenyl or pyrazolyl, which are optionally substituted, preferentially with one or more ($C_1$-$C_8$)alkyl, hydroxyl, (di)($C_1$-$C_8$)(alkyl)amino, ($C_1$-$C_8$)alkoxy or phenyl groups;

- **Ra** and **Rb**, which may be identical or different, represent a hydrogen atom or a ($C_1$-$C_8$)alkyl group, which is optionally substituted, preferentially with a hydroxyl group;
  or else the substituent Ra with a substituent of Het+ and/or Rb with a substituent of Ar form, together with the atoms that bear them, a (hetero)cycloalkyl; in particular, Ra and Rb represent a hydrogen atom or a ($C_1$-$C_4$)alkyl group optionally substituted with a hydroxyl group;

- **Q-** represents an organic or mineral anionic counterion, such as a halide or an alkyl sulfate.

In particular, mention may be made of the azo and hydrazono direct dyes bearing an endocyclic cationic charge of formulae (V) to (VIII) as defined previously, more particularly, the cationic direct dyes bearing an endocyclic cationic charge described in patent applications WO 95/15144, WO 95/01772 and EP 714 954, preferentially the following direct dyes:

[Chem. 17]

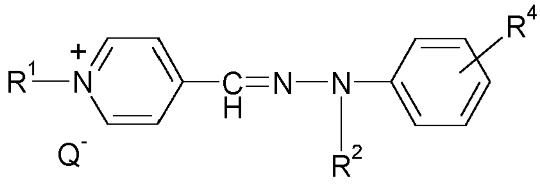

(IX)

[Chem. 18]

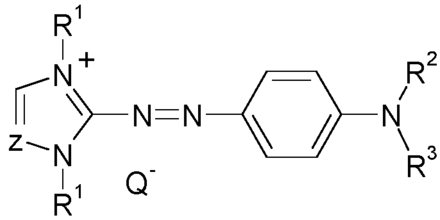

(X)

in which formulae (IX) and (X):

- **$R^1$** represents a ($C_1$-$C_4$)alkyl group such as methyl;
- **$R^2$** and **$R^3$**, which may be identical or different, represent a hydrogen atom or a ($C_1$-$C_4$)alkyl group, such as methyl; and
- **$R^4$** represents a hydrogen atom or an electron-donating group such as optionally substituted ($C_1$-$C_8$)alkyl, optionally substituted (Ci-Cs)alkoxy, or (di)($C_1$-$C_8$)(alkyl)amino optionally substituted on the alkyl group(s) with a hydroxyl group; particularly, $R^4$ is a hydrogen atom,
- **Z** represents a CH group or a nitrogen atom, preferentially CH,
- **Q-** is an anionic counterion as defined previously, in particular a halide, such as chloride, or an alkyl sulfate, such as methyl sulfate or mesyl.

In particular, the dyes of formulae (IX) and (X) are chosen from Basic Red 51, Basic Yellow 87 and Basic Orange 31 or derivatives thereof with Q' being an anionic counterion as defined previously, particularly a halide such as chloride, or an alkyl sulfate such as methyl sulfate or mesyl.

The direct dyes may be chosen from anionic direct dyes. The anionic direct dyes of the invention are dyes commonly referred to as "acid" direct dyes owing to their affinity for alkaline substances. The term "anionic direct dye" means any direct dye including in its structure at least one $CO_2R$ or $SO_3R$ substituent with R denoting a hydrogen atom or a cation originating from a metal or an amine, or an ammonium ion. The anionic dyes may be chosen from direct nitro acid dyes, azo acid dyes, azine acid dyes, triarylmethane acid dyes, indoamine acid dyes, anthraquinone acid dyes, indigoid dyes and natural acid dyes.

As acid dyes according to the invention, mention may be made of the dyes of formulae (XI), (XI'), (XII), (XII'), (XIII), (XIII'), (XIV), (XIV), (XV), (XVI), (XVII) and (XVIII) below:

a) the diaryl anionic azo dyes of formula (XI) or (XI'):

[Chem. 19]

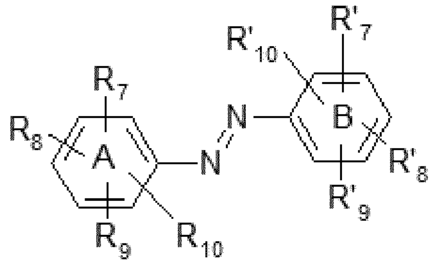

(XI)

[Chem. 20]

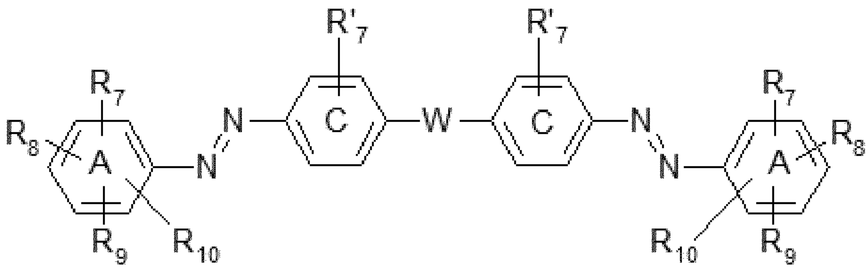

(XI')

in which formulae (XI) and (XI'):

- **$R_7$, $R_8$, $R_9$, $R_{10}$, $R'_7$, $R'_8$, $R'_9$** and **$R'_{10}$**, which may be identical or different, represent a hydrogen atom or a group chosen from:

  - alkyl;
  - alkoxy, alkylthio;
  - hydroxyl, mercapto;
  - nitro, nitroso;
  - R°-C(X)-X'-, R°-X'-C(X)-, R°-X'-C(X)-X"- with R° representing a hydrogen atom or an alkyl or aryl group; X, X' and X", which may be identical or different, representing an oxygen or sulfur atom, or NR with R representing a hydrogen atom or an alkyl group;
  - $(O)_2S(O^-)$-, M+ with M+ representing a hydrogen atom or a cationic counterion;
  - $(O)CO^-$-, M+ with M+ as defined previously;
  - R"-$S(O)_2$-, with R" representing a hydrogen atom or an alkyl, aryl, (di)(alkyl)amino or aryl(alkyl)amino group; preferentially a phenylamino or phenyl group;
  - R‴-$S(O)_2$-X'- with R‴ representing an optionally substituted alkyl or aryl group, X' as defined previously;
  - (di)(alkyl)amino;
  - aryl(alkyl)amino optionally substituted with one or more groups chosen from i) nitro; ii) nitroso; iii) $(O)_2S(O^-)$-, M+ and iv) alkoxy with $M^+$ as defined previously;

- optionally substituted heteroaryl; preferentially a benzothiazolyl group;

- cycloalkyl; notably cyclohexyl;

- Ar-N=N- with Ar representing an optionally substituted aryl group, preferentially a phenyl optionally substituted with one or more alkyl, $(O)_2S(O\text{-})\text{-}$, M+ or phenylamino groups;

- or alternatively two contiguous groups $R_7$ with $R_8$ or $R_8$ with $R_9$ or $R_9$ with $R_{10}$ together form a fused benzo group A'; and $R'_7$ with $R'_8$ or $R'_8$ with $R'_9$ or $R'_9$ with $R'_{10}$ together form a fused benzo group B'; with A' and B' optionally substituted with one or more groups chosen from i) nitro; ii) nitroso; iii) $(O)_2S(O\text{-})\text{-}$, M+; iv) hydroxyl; v) mercapto; vi) (di)(alkyl)amino; vii) R°-C(X)-X'-; viii) R°-X'-C(X)-; ix) R°-X'-C(X)-X"-; x) Ar-N=N- and xi) optionally substituted aryl(alkyl)amino; with M+, R°, X, X', X" and Ar as defined previously;

- **W** represents a sigma bond $\sigma$, an oxygen or sulfur atom, or a divalent radical i) - NR- with R as defined previously, or ii) methylene -C(Ra)(Rb)- with Ra and Rb, which may be identical or different, representing a hydrogen atom or an aryl group, or alternatively Ra and Rb form, with the carbon atom that bears them, a spiro cycloalkyl; preferentially, W represents a sulfur atom or Ra and Rb together form a cyclohexyl;

it being understood that formulae (XI) and (XI') comprise at least one sulfonate radical $(O)_2S(O\text{-})\text{-}$, M+ or one carboxylate radical $(O)CO^{-}\text{-}$, M+ on one of the rings A, A', B, B' or C; preferentially sodium sulfonate;

As examples of dyes of formula (XI), mention may be made of Acid Red 1, Acid Red 4, Acid Red 13, Acid Red 14, Acid Red 18, Acid Red 27, Acid Red 28, Acid Red 32, Acid Red 33, Acid Red 35, Acid Red 37, Acid Red 40, Acid Red 41, Acid Red 42, Acid Red 44, Pigment red 57, Acid Red 68, Acid Red 73, Acid Red 135, Acid Red 138, Acid Red 184, Food Red 1, Food Red 13, Acid Orange 6, Acid Orange 7, Acid Orange 10, Acid Orange 19, Acid Orange 20, Acid Orange 24, Yellow 6, Acid Yellow 9, Acid Yellow 36, Acid Yellow 199, Food Yellow 3, Acid Violet 7, Acid Violet 14, Acid Blue 113, Acid Blue 117, Acid Black 1, Acid Brown 4, Acid Brown 20, Acid Black 26, Acid Black 52, Food Black 1, Food Black 2, Food Yellow 3 or Sunset Yellow;

and as examples of dyes of formula (XI'), mention may be made of: Acid Red 111, Acid Red 134, Acid yellow 38.

b) the pyrazolone anionic azo dyes of formulae (XII) and (XII'):

[Chem. 21]

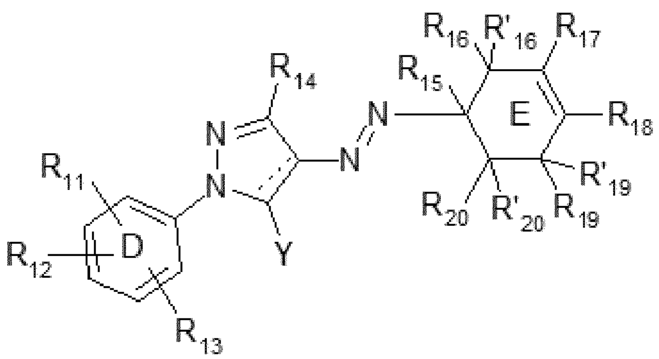

(XII)

[Chem. 22]

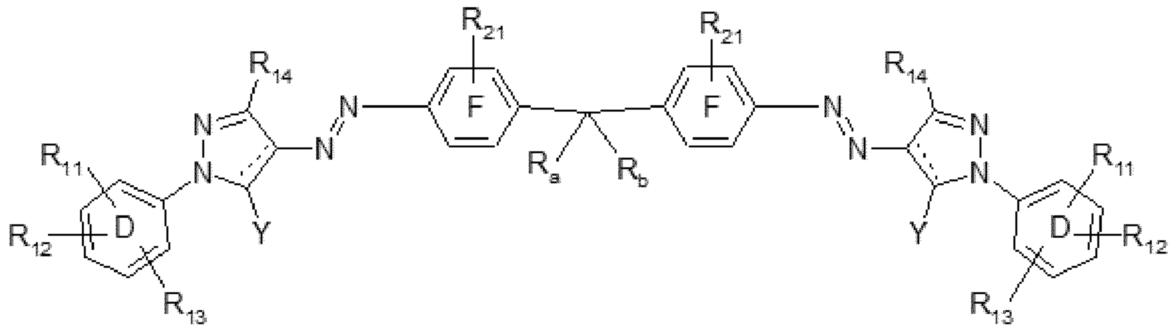

(XII')

in which formulae (XII) and (XII'):

- **Rn, $R_{12}$** and **$R_{13}$,** which may be identical or different, represent a hydrogen or halogen atom, an alkyl group or $-(O)_2S(O-)$, M+ with M+ as defined previously;
- **$R_{14}$** represents a hydrogen atom, an alkyl group or a group -C(O)O-, M+ with M+ as defined previously;
- **$R_{15}$** represents a hydrogen atom;
- **$R_{16}$** represents an oxo group, in which case $R'_{16}$ is absent, or alternatively **$R_{15}$** with **$R_{16}$** together form a double bond;
- **$R_{17}$** and **$R_{18}$,** which may be identical or different, represent a hydrogen atom, or a group chosen from:
  - $(O)_2S(O-)-$, M+ with M+ as defined previously;
  - $Ar-O-S(O)_2-$ with Ar representing an optionally substituted aryl group; preferentially a phenyl optionally substituted with one or more alkyl groups;
  - **$R_{19}$** and **$R_{20}$** together form either a double bond, or a benzo group D', which is optionally substituted;
  - **$R'_{16}$, $R'_{19}$** and **$R'_{20}$,** which may be identical or different, represent a hydrogen atom or an alkyl or hydroxyl group;
  - **$R_{21}$** represents a hydrogen atom or an alkyl or alkoxy group;
  - **$R_a$** and **$R_b$,** which may be identical or different, are as defined previously, preferentially $R_a$ represents a hydrogen atom and $R_b$ represents an aryl group;
  - **Y** represents either a hydroxyl group or an oxo group;
  - 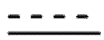

    represents a single bond when Y is an oxo group; and represents a double bond when Y represents a hydroxyl group;

it being understood that formulae (XII) and (XII') comprise at least one sulfonate radical $(O)_2S(O-)-$, M+ or one carboxylate radical -C(O)O-, M+ on one of the rings D or E; preferentially sodium sulfonate;

As examples of dyes of formula (XII), mention may be made of Acid Red 195, Acid Yellow 23, Acid Yellow 27, Acid Yellow 76, and as examples of dyes of formula (XII'), mention may be made of: Acid Yellow 17;

c) the anthraquinone dyes of formulae (XIII) and (XIII'):

[Chem. 23]

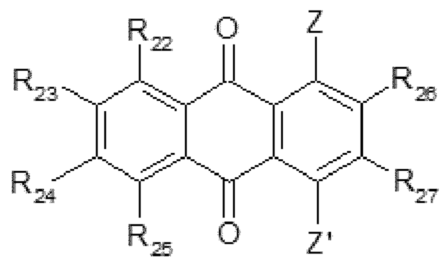

(XIII)

[Chem. 24]

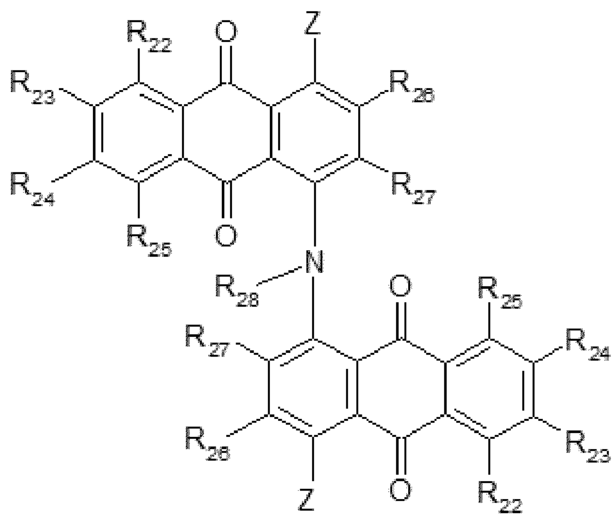

(XIII')

in which formulae (XIII) and (XIII'):

- $R_{22}$, $R_{23}$, $R_{24}$, $R_{25}$, $R_{26}$ and $R_{27}$, which may be identical or different, represent a hydrogen or halogen atom, or a group chosen from:

- alkyl;
- hydroxyl, mercapto;
- alkoxy, alkylthio;
- optionally substituted aryloxy or arylthio, preferentially substituted with one or more groups chosen from alkyl and $(O)_2S(O^-)$-, M+ with M+ as defined previously;
- aryl(alkyl)amino optionally substituted with one or more groups chosen from alkyl and $(O)_2S(O^-)$-, M+ with M+ as defined previously;
- (di)(alkyl)amino;

- (di)(hydroxyalkyl)amino;

- $(O)_2S(O-)-$, M+ with M+ as defined previously;

Z' represents a hydrogen atom or a group NR28R29 with R28 and R29, which may be identical or different, representing a hydrogen atom or a group chosen from:

- alkyl;

- polyhydroxyalkyl such as hydroxyethyl;

- aryl optionally substituted with one or more groups, particularly i) alkyl such as methyl, n-dodecyl, n-butyl; ii) $(O)_2S(O-)-$, M+ with M+ as defined previously; iii) R°-C(X)-X'-, R°-X'-C(X)-, R°-X'-C(X)-X"- with R°, X, X' and X" as defined previously, preferentially R° represents an alkyl group;

- cycloalkyl; notably cyclohexyl;

Z, represents a group chosen from hydroxyl and NR'28R'29 with R'28 and R'29, which may be identical or different, representing the same atoms or groups as R28 and R29 as defined previously;

it being understood that formulae (XIII) and (XIII') comprise at least one sulfonate radical $(O)_2S(O-)-$, M+ or one carboxylate radical C(O)O-, M+; preferentially sodium sulfonate;

As examples of dyes of formula (XIII), mention may be made of Acid Blue 25, Acid Blue 43, Acid Blue 62, Acid Blue 78, Acid Blue 129, Acid Blue 138, Acid Blue 140, Acid Blue 251, Acid Green 25, Acid Green 41, Acid Violet 42, Acid Violet 43, Mordant Red 3; EXT violet No. 2; and, as an example of a dye of formula (XIII'), mention may be made of: Acid Black 48;

d) the nitro dyes of formulae (XIV) and (XIV'):

[Chem. 25]

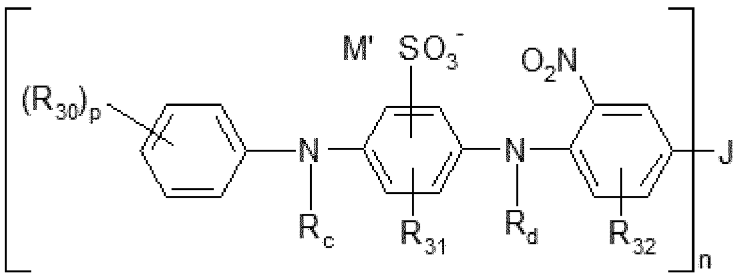

(XIV)

[Chem. 26]

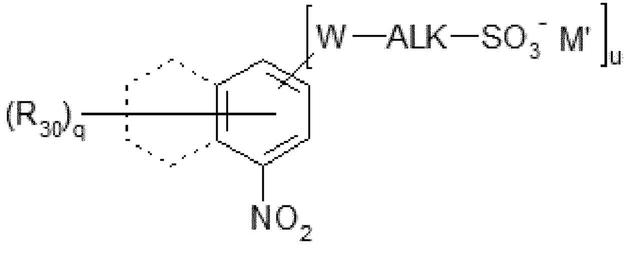

(XIV')

in which formulae (XIV) and (XIV):

- $R_{30}$, $R_{31}$ and $R_{32}$, which may be identical or different, represent a hydrogen or halogen atom, or a group chosen from:
  - alkyl;
  - alkoxy optionally substituted with one or more hydroxyl groups, alkylthio optionally substituted with one or more hydroxyl groups;
  - hydroxyl, mercapto;
  - nitro, nitroso;
  - polyhaloalkyl;
  - R°-C(X)-X'-, R°-X'-C(X)-, R°-X'-C(X)-X"- with R°, X, X' and X" as defined previously;
  - $(O)_2S(O-)-$, M+ with M+ as defined previously;
  - (O)CO--, M+ with M+ as defined previously;
  - (di)(alkyl)amino;
  - (di)(hydroxyalkyl)amino;
  - heterocycloalkyl such as piperidino, piperazino or morpholino; in particular, $R_{30}$, $R_{31}$ and $R_{32}$ represent a hydrogen atom;
  - Rc and Rd, which may be identical or different, represent a hydrogen atom or an alkyl group;
  - W is as defined previously; W particularly represents an -NH- group;
  - ALK represents a linear or branched divalent $C_1$-$C_6$ alkylene group; in particular, ALK represents a $-CH_2-CH_2-$ group;
  - n is 1 or 2;
  - p represents an integer inclusively between 1 and 5;
  - q represents an integer inclusively between 1 and 4;
  - u is 0 or 1;
  - when n is 1, J represents a nitro or nitroso group; particularly nitro;
  - when n is 2, J represents an oxygen or sulfur atom, or a divalent radical -S(O)m- with m representing an integer 1 or 2; preferentially, J represents an -SO2- radical;
  - M' represents a hydrogen atom or a cationic counterion;
  - 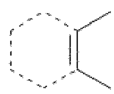,

    which may be present or absent, represents a benzo group optionally substituted with one or more groups R30 as defined previously;

it being understood that formulae (XIV) and (XIV') comprise at least one sulfonate radical $(O)_2S(O-)$-, M+ or one carboxylate radical -C(O)O-, M+; preferentially sodium sulfonate.

As examples of dyes of formula (XIV), mention may be made of Acid Brown 13 and Acid Orange 3; as examples of dyes of formula (XIV), mention may be made of: Acid Yellow 1, the sodium salt of 2,4-dinitro-1-naphthol-7-sulfonic acid, 2-piperidino-5-nitrobenzenesulfonic acid, 2-(4'-N,N-(2"-hydroxyethyl)amino-2'-nitro)anilineethanesulfonic acid, 4-β-hydroxyethylamino-3-nitrobenzenesulfonic acid; EXT D&C Yellow 7;

e) the triarylmethane dyes of formula (XV):

[Chem. 27]

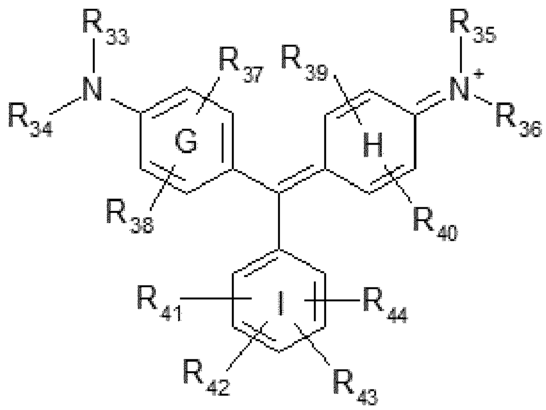

(XV)

in which formula (XV):

- $R_{33}$, $R_{34}$, $R_{35}$ and $R_{36}$, which may be identical or different, represent a hydrogen atom or a group chosen from alkyl, optionally substituted aryl and optionally substituted arylalkyl; particularly an alkyl and benzyl group optionally substituted with a group $(O)_mS(O-)$-, M+ with M+ and m as defined previously;
- $R_{37}$, $R_{38}$, $R_{39}$, $R_{40}$, $R_{41}$, $R_{42}$, $R_{43}$ and $R_{44}$, which may be identical or different, represent a hydrogen atom or a group chosen from:
  - alkyl;
  - alkoxy, alkylthio;
  - (di)(alkyl)amino;
  - hydroxyl, mercapto;
  - nitro, nitroso;
  - R°-C(X)-X'-, R°-X'-C(X)-, R°-X'-C(X)-X"- with R° representing a hydrogen atom or an alkyl or aryl group; X, X' and X", which may be identical or different, representing an oxygen or sulfur atom, or NR with R representing a hydrogen atom or an alkyl group;
  - $(O)_2S(O-)$-, M+ with M+ representing a hydrogen atom or a cationic counterion;
  - (O)CO--, M+ with M+ as defined previously;
  - or alternatively two contiguous groups $R_{41}$ with $R_{42}$ or $R_{42}$ with $R_{43}$ or $R_{43}$ with $R_{44}$ together form a fused benzo group: I'; with I' optionally substituted with one or more groups chosen from i) nitro; ii) nitroso; iii) $(O)_2S(O-)$-, M+; iv) hydroxyl; v) mercapto; vi) (di)(alkyl)amino; vii) R°-C(X)-X'-; viii) R°-X'-C(X)- and ix) R°-X'-C(X)-X"-; with M+, R°, X, X' and X" as defined previously;

in particular, $R_{37}$ to $R_{40}$ represent a hydrogen atom, and $R_{41}$ to $R_{44}$, which may be identical or different, represent a hydroxyl group or $(O)_2S(O\text{-})\text{-}$, M+; and when $R_{43}$ with $R_{44}$ together form a benzo group, it is preferentially substituted with an $(O)_2S(O\text{-})\text{-}$ group;

it being understood that at least one of the rings G, H, I or I' comprises at least one sulfonate radical $(O)_2S(O^-)\text{-}$ or a carboxylate radical -C(O)O-; preferentially sulfonate;

As examples of dyes of formula (XV), mention may be made of: Acid Blue 1; Acid Blue 3; Acid Blue 7, Acid Blue 9; Acid Violet 49; Acid Green 3; Acid Green 5 and Acid Green 50;

f) the xanthene-based dyes of formula (XVI):

[Chem. 28]

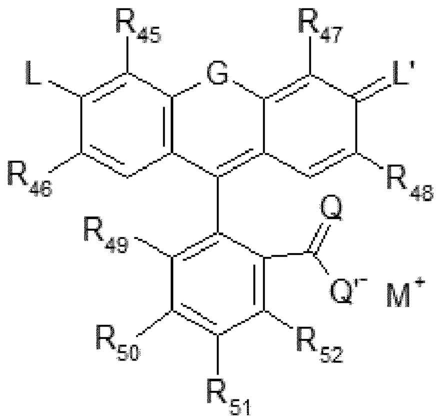

(XVI)

in which formula (XVI):

- $R_{45}$, $R_{46}$, $R_{47}$ and $R_{48}$, which may be identical or different, represent a hydrogen or halogen atom;
- $R_{49}$, $R_{50}$, $R_{51}$ and $R_{52}$, which may be identical or different, represent a hydrogen or halogen atom, or a group chosen from:
    - alkyl;
    - alkoxy, alkylthio;
    - hydroxyl, mercapto;
    - nitro, nitroso;
    - $(O)_2S(O\text{-})\text{-}$, M+ with M+ representing a hydrogen atom or a cationic counterion;
    - (O)CO--, M+ with M+ as defined previously;

particularly, $R_{53}$, $R_{54}$, $R_{55}$ and $R_{48}$ represent a hydrogen or halogen atom;
**[0146]** - G represents an oxygen or sulfur atom or a group NRe with Re as defined previously; particularly G represents an oxygen atom;

- L represents an alkoxide O-, M+; a thioalkoxide S-, M+ or a group NRf, with Rf representing a hydrogen atom or an alkyl group, and M+ as defined previously; M+ is particularly sodium or potassium;

- L' represents an oxygen or sulfur atom or an ammonium group: N+RfRg, with Rf and Rg, which may be identical or different, representing a hydrogen atom or an optionally substituted alkyl or aryl group; L' particularly represents an oxygen atom or a phenylamino group optionally substituted with one or more alkyl or $(O)_mS(O\text{-})\text{-}$, M+ groups with m and M+ as defined previously;

- Q and Q', which may be identical or different, represent an oxygen or sulfur atom; in particular, Q and Q' represent an oxygen atom;

- M+ is as defined previously;

As examples of dyes of formula (XVI), mention may be made of Acid Yellow 73; Acid Red 51; Acid Red 52; Acid Red 87; Acid Red 92; Acid Red 95; Acid Violet 9;

g) the indole-based dyes of formula (XVII):

[Chem. 29]

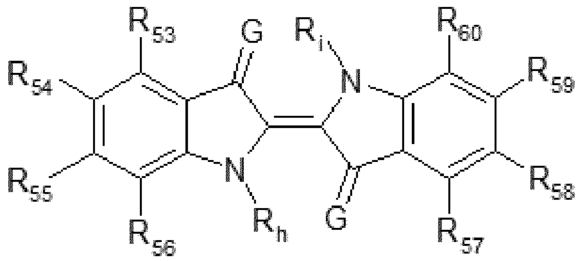

(XVII)

$R_{53}$, $R_{54}$, $R_{55}$, $R_{56}$, $R_{57}$, $R_{58}$, $R_{59}$ and $R_{60}$, which may be identical or different, represent a hydrogen atom or a group chosen from:

- alkyl;

- alkoxy, alkylthio;

- hydroxyl, mercapto;

- nitro, nitroso;

- R°-C(X)-X'-, R°-X'-C(X)-, R°-X'-C(X)-X"- with R° representing a hydrogen atom or an alkyl or aryl group; X, X' and X", which may be identical or different, representing an oxygen or sulfur atom, or NR with R representing a hydrogen atom or an alkyl group;

- $(O)_2S(O\text{-})\text{-}$, M+ with M+ representing a hydrogen atom or a cationic counterion;

- (O)CO--, M+ with M+ as defined previously;

- G represents an oxygen or sulfur atom or a group NRe with Re as defined previously; in particular, G represents an oxygen atom;

- Ri and Rh, which may be identical or different, represent a hydrogen atom or an alkyl group; it being understood that formula (XVII) comprises at least one sulfonate radical $(O)_2S(O\text{-})\text{-}$, M+ or one carboxylate radical -C(O)O-, M+; preferentially sodium sulfonate.

As an example of dyes of formula (XVII), mention may be made of: Acid Blue 74.
h) the quinoline-based dyes of formula (XVIII):

[Chem. 30]

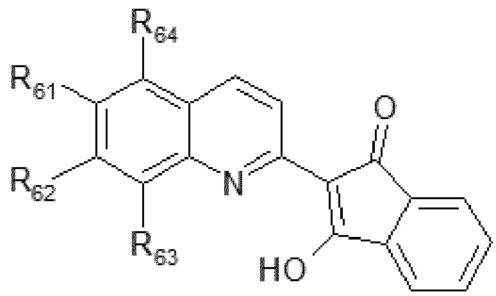

(XVIII)

- $R_{61}$ represents a hydrogen or halogen atom or an alkyl group;
- $R_{62}$, $R_{63}$ and $R_{64}$, which may be identical or different, represent a hydrogen atom or a group $(O)_2S(O-)$-, M+ with M+ representing a hydrogen atom or a cationic counterion;

or alternatively $R_{61}$ with $R_{62}$, or $R_{61}$ with $R_{64}$, together form a benzo group optionally substituted with one or more groups $(O)_2S(O-)$-, M+ with M+ representing a hydrogen atom or a cationic counterion;

it being understood that formula (XVIII) comprises at least one sulfonate radical $(O)_2S(O-)$-, M+ preferentially sodium sulfonate.

As examples of dyes of formula (XVIII), mention may be made of: Acid Yellow 2, Acid Yellow 3 and Acid Yellow 5.

Among the natural direct dyes that may be used according to the invention, mention may be made of lawsone, juglone, alizarin, purpurin, carminic acid, kermesic acid, purpurogallin, protocatechaldehyde, indigo, isatin, curcumin, spinulosin, apigenidin and orceins. Use may also be made of extracts or decoctions comprising these natural dyes and in particular henna-based poultices or extracts.

Preferably, the direct dye(s) are chosen from anionic direct dyes.

The colouring agent(s) may be present in a total amount ranging from 0.001% to 15% by weight and preferably from 0.005% to 10% by weight relative to the total weight of composition (C).

The pigments may be present in concentrations ranging from 0.05% to 15% by weight, preferably from 0.1% to 10% by weight, relative to the total weight of the composition.

The direct dye(s) may be present in concentrations ranging from 0.001% to 10% by weight of the total weight of the composition, preferably from 0.005% to 5% by weight.

Composition (C) according to the invention comprises water. Preferably, water is present in a content ranging from 0.1% to 50% by weight, more preferentially from 0.5% to 30% by weight, relative to the total weight of the composition.

Composition (C) according to the invention may comprise less than 2% by weight of water relative to the total weight of the dye composition.

**Solvents:**

Composition (C) according to the invention may comprise one or more organic solvents.

Examples of organic solvents that may be mentioned include lower $C_1$-$C_4$ alkanols, such as ethanol and isopropanol; polyols and polyol ethers, for instance 2-butoxyethanol, propylene glycol, propylene glycol monomethyl ether and diethylene glycol monoethyl ether and monomethyl ether, and also aromatic alcohols, for instance benzyl alcohol or phenoxyethanol, and mixtures thereof.

Preferably, composition (C) comprises one or more organic solvents chosen from $C_1$-$C_4$ lower alkanols, more preferentially ethanol.

The organic solvents may be present in a total amount inclusively between 0.1% and 20% by weight approximately relative to the total weight of the dye composition, preferably between 0.5% and 15% by weight and more preferentially inclusively between 1% and 15% by weight relative to the total weight of the composition.

According to a particular embodiment, the organic solvent(s) may be present in a total amount inclusively between 0.5% and 80% by weight relative to the total weight of composition (C), preferably between 1% and 70% by weight, more preferentially inclusively between 5% and 60% and better still between 10% and 50% by weight relative

to the total weight of composition (C).

**Additives:**

Composition (C) may also contain any adjuvant or additive usually used.

Among the additives that may be contained in the composition, mention may be made of reducing agents, thickeners, softeners, antifoams, moisturizers, UV-screening agents, peptizers, solubilizers, fragrances, anionic, cationic, nonionic or amphoteric surfactants, proteins, vitamins, polymers, preserving agents, oils, waxes and mixtures thereof.

Composition (C) according to the invention may notably be in the form of a suspension, a dispersion, a gel, an emulsion, notably an oil-in-water (O/W) or water-in-oil (W/O) emulsion, or a multiple emulsion (W/O/W or polyol/O/W or O/W/O), in the form of a cream, a mousse, a stick, a dispersion of vesicles, notably of ionic or nonionic lipids, or a two-phase or multi-phase lotion.

In a preferred embodiment, the cosmetic composition according to the invention is an aqueous and/or aqueous-alcoholic system or a thickened emulsion in water.

The thickener(s) may be chosen from mineral or organic thickeners. The thickener may be nonionic, cationic, anionic or amphoteric.

The thickener(s) may notably be chosen from carboxyvinyl polymers such as crosslinked acrylic acid homopolymers (carbomer) such as those sold under the name Carbopol by the company Goodrich, polyacrylates and polymethacrylates such as the products sold under the names Lubrajel or Norgel 25 by the company Guardian or under the name Hispagel by the company Hispano Chimica; polyacrylamides such as the product sold under the name Sepigel 305 by the company SEPPIC; polysaccharides such as alginates, cellulose and derivatives thereof, notably carboxymethylcellulose, hydroxymethylcellulose, hydroxypropylcellulose and microcrystalline cellulose; natural gums such as xanthan gum, guar gum, locust bean gum, acacia gum, scleroglucans, chitin and chitosan derivatives, carrageenans; or clays such as montmorillonite and derivatives, bentones and derivatives, and magnesium aluminium silicates and derivatives (Veegum).

According to a particular embodiment of the invention, composition (C) comprises at least one thickener chosen from crosslinked acrylic and/or methacrylic acid polymers.

According to a particular embodiment of the invention, the composition comprises at least one thickener chosen from crosslinked acrylic acid homopolymers.

The thickener may be present in the composition in a total content ranging from 0.01% to 10% by weight relative to the weight of the composition, preferably from 0.1% to 5% by weight relative to the weight of the composition, preferably from 0.4% to 2% by weight relative to the weight of the composition.

A person skilled in the art may select the appropriate presentation form, and also the method for preparing it, on the basis of his or her general knowledge, taking into account firstly the nature of the constituents used, notably their solubility in the support, and secondly the intended application of the composition.

According to a preferred embodiment, composition (C) according to the invention comprises 3-aminopropyltriethoxysilane (APTES), at least one non-amino silicone of formula (III) as described previously, and at least one pigment.

According to a more preferred embodiment, composition (C) according to the invention comprises 3-aminopropyltriethoxysilane (APTES), at least one non-amino silicone of formula (IIIa) or (IIId)as described previously, and at least one pigment.

The present invention also relates to the use of composition (C) as described above for the cosmetic treatment of, in particular for dyeing, keratin fibres, in particular human keratin fibres such as the hair.

**Process for treating keratin fibres:**

The present invention also relates to a process for treating human keratin fibres such as the hair, which consists in extemporaneously mixing, at the time of use, two compositions (A) and (B) and in applying the mixture to the fibres, said mixture comprising an alkoxysilane/non-amino silicone mass ratio ranging from 95/5 to 5/95, with:

- composition (A) comprising at least one alkoxysilane chosen from the compounds of formula (I), oligomers thereof and/or mixtures thereof as described previously;

- composition (B) comprising at least one non-amino silicone chosen from the compounds of formula (III) as described previously; and at least one colouring agent chosen from pigments, direct dyes and mixtures thereof; it being understood that composition (A) and/or composition (B) comprise water.

Preferably, composition (A) does not comprise at least one non-amino silicone chosen from the compounds of formula (III) and composition (B) does not comprise at least one alkoxysilane chosen from the compounds of formula

(I), oligomers thereof and/or mixtures thereof.

According to a first embodiment, water is present in composition (A). Thus, composition (A) and composition (B) are mixed extemporaneously at the time of use before being applied to the keratin fibres.

According to a second embodiment, water is present in composition (B). Thus, composition (A) and composition (B) are mixed extemporaneously at the time of use before being applied to the keratin fibres.

According to a third embodiment, water is present in a composition (D). According to this third embodiment, composition (A), composition (B) and composition (D) are mixed extemporaneously at the time of use before being applied to the keratin fibres.

According to another particular embodiment, the process of the invention is a process for treating human keratin fibres such as the hair, which consists in extemporaneously mixing, at the time of use, two compositions (A) and (B) and in applying the mixture to the fibres moistened with water, said mixture comprising an alkoxysilane/non-amino silicone mass ratio ranging from 95/5 to 5/95, with:

- composition (A) comprising at least one alkoxysilane chosen from the compounds of formula (I), oligomers thereof and/or mixtures thereof as described previously; and
- composition (B) comprising at least one non-amino silicone chosen from the compounds of formula (III) as described previously; and at least one colouring agent chosen from pigments, direct dyes and mixtures thereof.

Thus, composition (C) applied to the keratin fibres is obtained by extemporaneous mixing of compositions (A) and (B) or extemporaneous mixing of compositions (A), (B) and (D).

Preferably, the alkoxysilane of formula (I)/non-amino silicone of formula (III) mass ratio ranges from 90/10 to 10/90, preferably from 60/40 to 40/60; more preferentially, the alkoxysilane of formula (I)/non-amino silicone of formula (III) mass ratio is 50/50.

Preferably, composition (A) and/or composition (B) comprises one or more organic solvents, better still chosen from $C_1$-$C_4$ lower alkanols, more preferentially ethanol.

The present invention also relates to a process for dyeing keratin fibres, in particular human keratin fibres, preferably the hair, comprising:

- the application to said fibres of a composition comprising:

  a) at least one alkoxysilane chosen from the compounds of formula (I), oligomers thereof and/or mixtures thereof as described previously;
  b) at least one non-amino silicone chosen from the compounds of formula (III) as described previously;
  c) water and
  d) at least one colouring agent chosen from pigments, direct dyes and mixtures thereof as described previously.

Preferably, the alkoxysilane of formula (I)/non-amino silicone of formula (III) mass ratio ranges from 95/5 to 5/95, better still from 90/10 to 10/90, more preferentially from 60/40 to 40/60; even more preferentially, the alkoxysilane of formula (I)/non-amino silicone of formula (III) mass ratio is 50/50.

According to a particular embodiment, the present invention also relates to a process for treating human keratin fibres such as the hair, which consists in extemporaneously mixing, at the time of use, two compositions (A) and (B) and in applying said mixture to the fibres, with:

- composition (A) comprising at least one alkoxysilane chosen from the compounds of formula (Ia) as described previously, oligomers thereof and/or mixtures thereof;
- composition (B) comprising at least one non-amino silicone of formula (IIId) as described previously;

it being understood that composition (A) and/or composition (B) comprise water, and it being understood that composition (A) and/or composition (B) comprise at least one colouring agent chosen from pigments, direct dyes and mixtures thereof.

Preferably, composition (A) does not comprise at least one non-amino silicone of formula (IIId) and composition (B) does not comprise at least one alkoxysilane chosen from the compounds of formula (Ia), oligomers thereof and/or mixtures thereof.

According to a first embodiment, water is present in composition (A). The colouring agent is present in composition (B). Thus, composition (A) and composition (B) are mixed extemporaneously at the time of use before being applied to the keratin fibres.

According to a second embodiment, water is present in composition (B). The colouring agent is present in composition (A). Thus, composition (A) and composition (B) are mixed extemporaneously at the time of use before being applied to the keratin fibres.

According to a third embodiment, water and the colouring agent are present in composition (D). Thus, composition (A), composition (B) and composition (D) are mixed extemporaneously at the time of use before being applied to the keratin fibres.
Preferably, composition (A) comprises water and composition (B) comprises at least one colouring agent chosen from pigments, direct dyes and mixtures thereof.
According to another particular embodiment, the process of the invention is a process for treating keratin fibres such as the hair, which consists in extemporaneously mixing, at the time of use, two compositions (A) and (B) and in applying the mixture to the fibres moistened with water, with:

- composition (A) comprising at least one alkoxysilane chosen from the compounds of formula (Ia) as described previously, oligomers thereof and/or mixtures thereof;

- composition (B) comprising at least one non-amino silicone of formula (IIId) as described previously;

it being understood that composition (A) and/or composition (B) comprise at least one colouring agent chosen from pigments, direct dyes and mixtures thereof.
Thus, composition (C) applied to the keratin fibres is obtained by extemporaneous mixing of compositions (A) and (B) or extemporaneous mixing of compositions (A), (B) and (D).
The cosmetic composition described above may be used on wet or dry keratin fibres, and also on any type of fair or dark, natural or dyed, permanent-waved, bleached or relaxed fibres.
According to a particular embodiment of the invention, the fibres are washed before applying the cosmetic composition (C).
The application to the fibres may be performed via any standard means, in particular using a comb, a fine brush, a coarse brush, a sponge or with the fingers.
Preferably, after applying the cosmetic composition (C) to the keratin fibres, there is a waiting time of between 1 minute and 6 hours, in particular between 10 minutes and 5 hours, more particularly between 30 minutes and 4 hours, more preferentially about 1 hour.
Preferably, after applying the cosmetic composition (C) to the keratin fibres, the fibres are left to dry or are dried, for example at a temperature of greater than or equal to 30°C.
According to a particular embodiment, the fibres may be dried at a temperature of greater than or equal to 40°C. According to a particular embodiment, the fibres may be dried at a temperature of greater than 40°C and less than 100°C.
Preferably, if the fibres are dried, they are dried, in addition to a supply of heat, with a flow of air.
During drying, a mechanical action may be exerted on the locks, such as combing, brushing or running the fingers through. This operation may similarly be performed once the fibres have been dried, naturally or otherwise.
The drying step may be performed with a drying device such as a hood, a hairdryer, a climazone, etc.
When the drying step is performed with a hood or a hairdryer, the drying temperature is between 40 and 110°C, preferably between 50 and 90°C.
After the drying step, a shaping step may be performed, for example with a straightening iron; the temperature for the shaping step is between 110 and 220°C, preferably between 140 and 200°C.
Once the drying is complete, final rinsing or shampooing may optionally be performed.
According to a particular variant of the invention, a step of acidic rinsing at a pH of between 1 and 5 may be applied after the step of drying the locks and before the final shampoo wash.
According to a particular variant of the invention, a care of mask type with a pH of between 1 and 5 may be applied after the step of drying the locks and before the final shampoo wash with a leave-on time of between 1 and 45 minutes, in particular between 5 minutes and 30 minutes and preferably about 15 minutes.
According to a preferred embodiment, the process of the invention is a process for dyeing keratin fibres, in particular human keratin fibres, preferably the hair, comprising the application to said fibres of a composition (C) comprising:

a) at least one alkoxysilane chosen from the compounds of formula (I), oligomers thereof and/or mixtures thereof as described previously;
b) at least one non-amino silicone chosen from the compounds of formula (III) as described previously;
c) water and
d) at least one colouring agent chosen from pigments, direct dyes and mixtures thereof as described previously; and

followed by a drying step as defined previously and optionally followed by a step of rinsing at acidic pH as defined previously and/or a shampoo wash;

said dyeing process may be repeated several times, it being understood that each new application of composition (C) is performed after an interval ranging from 1 minute to several weeks.

Preferably, the alkoxysilane(s) chosen from the compounds of formula (I), oligomers thereof and/or a mixture thereof and the non-amino silicone(s) of formula (III) included in composition (C) are identical or different in each step of repetition of the dyeing process.

According to another preferred embodiment, the process is a process for dyeing keratin fibres, in particular human keratin fibres, preferably the hair, comprising:

1) the application to said fibres of a composition (E) comprising at least one colouring agent chosen from pigments, direct dyes and mixtures thereof as described previously; and
2) the application to said fibres of a composition (F) comprising:

    a) at least one alkoxysilane chosen from the compounds of formula (I), oligomers thereof and/or mixtures thereof as described previously;
    b) at least one non-amino silicone chosen from the compounds of formula (III) as described previously;
    c) at least water; and

followed by a drying step as defined previously and optionally followed by a step of rinsing at acidic pH as defined previously and/or a shampoo wash.

Preferably, composition (E) is a pigmented composition, which is preferably aqueous, aqueous-alcoholic, alcoholic or anhydrous, in spray, mascara, pen, roll-on or pad form. Such products are sold, for example, by L'Oreal Paris under the name Magic Retouch or by Garnier under the name Express Retouch.

Step 2, i.e. the step of applying composition (F) above, may be repeated several times.

According to a particular embodiment, the process is a process for dyeing keratin fibres, in particular human keratin fibres, preferably the hair, comprising the application to fibres moistened with water of a composition (C) comprising:

a) at least one alkoxysilane chosen from the compounds of formula (I), oligomers thereof and/or mixtures thereof as described previously;
b) at least one non-amino silicone chosen from the compounds of formula (III) as described previously;
c) at least one colouring agent chosen from pigments, direct dyes and mixtures thereof as described previously; and

followed by a drying step as defined previously and optionally followed by a step of rinsing at acidic pH as defined previously and/or a shampoo wash.

The present invention also relates to a device for treating human keratin fibres such as the hair, comprising two compartments containing:

- in a first compartment (C1), a composition (A) comprising at least one alkoxysilane chosen from the compounds of formula (I), oligomers thereof and/or mixtures thereof as described previously;
- in a second compartment (C2), a composition (B) comprising at least one non-amino silicone chosen from the compounds of formula (III) as described previously; and at least one colouring agent chosen from a pigment, a direct dye and mixtures thereof;

it being understood that composition (A) and/or composition (B) comprise water, and the mixing of the compositions of compartments (C1) and (C2) gives an alkoxysilane/non-amino silicone mass ratio ranging from 95/5 to 5/95.

According to a particular embodiment, the treating device according to the invention is a device for treating human keratin fibres such as the hair, comprising two compartments containing:

- in a first compartment (C1), a composition (A) comprising at least one alkoxysilane chosen from the compounds of formula (I), oligomers thereof and/or mixtures thereof as described previously; and at least one colouring agent chosen from pigments, direct dyes and mixtures thereof;
- in a second compartment (C2), a composition (B) comprising at least one non-amino silicone chosen from the compounds of formula (III) as described previously and at least one colouring agent chosen from pigments, direct dyes and mixtures thereof;

it being understood that composition (A) and/or composition (B) comprise water, and the mixing of the compositions of compartments (C1) and (C2) gives an alkoxysilane/non-amino silicone mass ratio ranging from 95/5 to 5/95.

According to a particular embodiment, the treating device a device for treating human keratin fibres such as the hair, comprising three compartments containing:

- in a first compartment (C1), a composition (A) comprising at least one alkoxysilane chosen from the compounds of formula (I), oligomers thereof and/or mixtures thereof as described previously; and at least one colouring agent chosen from pigments, direct dyes and mixtures thereof;
- in a second compartment (C2), a composition (B) comprising at least one non-amino silicone chosen from the compounds of formula (III) as described previously; and at least one colouring agent chosen from pigments, direct dyes and mixtures thereof; and
- in a third compartment (C3), a composition (D) comprising water;

it being understood that the mixing of the compositions in compartments (C1), (C2) and (C3) gives an alkoxysilane/non-amino silicone mass ratio ranging from 95/5 to 5/95.

According to a particular embodiment, the treating device according to the invention is a device for treating keratin fibres such as the hair, comprising two compartments containing:

- in a first compartment (C1), a composition (A) comprising at least one alkoxysilane chosen from the compounds of formula (Ia), oligomers thereof and/or mixtures thereof as described previously;
- in a second compartment (C2), a composition (B) comprising at least one non-amino silicone of formula (III d) as described previously;

  it being understood that composition (A) and/or composition (B) comprise water, and
  it being understood that composition (A) and/or composition (B) comprise at least one colouring agent chosen from pigments, direct dyes and mixtures thereof.

According to a particular embodiment, the treating device is a device for treating keratin fibres such as the hair, comprising at least three compartments containing:

- in a first compartment (C1), a composition (A) comprising at least one alkoxysilane chosen from the compounds of formula (Ia), oligomers thereof and/or mixtures thereof as described previously;
- in a second compartment (C2), a composition (B) comprising at least one non-amino silicone of formula (III d) as described previously; and
- in a third compartment (C3), a composition (D) comprising water;

it being understood that composition (A), composition (B) and/or composition (D) comprise at least one colouring agent chosen from pigments, direct dyes and mixtures thereof.

The present invention will now be described more specifically by means of examples, which do not in any way limit the scope of the invention. However, the examples make it possible to support specific features, variants and preferred embodiments of the invention.

**Example**

**Example 1: Use of APTES**

Solution 1 (i.e. solution of 3-aminopropyltriethoxysilane (APTES)) is prepared according to the process below:

- pure APTES (APTES Sil soft A-1100 sold by the company Momentive Performance Materials) is mixed with an aqueous solution brought to pH=1 by adding HCl, then a solution of ethanol is added and said mixture is placed on a VWR brand magnetic stirrer (rotation speed 500 rpm) for 24 hours at room temperature.

The alcoholic solution 2 (i.e. alcoholic solution of non-amino silicone) is prepared according to the process below:

- the poly dimethylsiloxane (PDMS) compound bearing hydroxyl end functions (PDMS reference 481939 sold by the company Sigma-Aldrich) is diluted in an ethanol solution in which the pigment (iron oxide sold by the company Sun Chemical under the name SunPuro Red Iron Oxide®) is dispersed.

Solution 1 is mixed with solution 2 and said mixture is applied to the keratin fibres.

The various compounds are present in the amounts below in g and also the mass ratio in the final APTES/PDMS

mixture.

[Tables 1]

| Chemical name | | Composition A | Composition B |
|---|---|---|---|
| Solution 1 | 3-Aminopropyltriethoxysilane (APTES) | 2 | 0.3 |
| | Ethanol | 0.89 | 0.13 |
| | Water | 0.12 | 0.02 |
| Solution 2 | Polydimethylsiloxanes (PDMS) bearing hydroxyl end functions | 1 | 2.7 |
| | Pigment (iron oxide sold by the company Sun Chemical under the name SunPuro Red Iron Oxide®) | 0.3 | 0.3 |
| | Ethanol | 7.70 | 8.55 |
| APTES/PDMS ratio | | 67/33 | 10/90 |

Protocol:

The cosmetic compositions (mixture of solution 1 and of solution 2 in the various ratios) are applied to locks of dry natural hair containing 90% white hairs, at a rate of 1 g of composition per gram of lock.
The locks of hair are then combed and dried with a hairdryer.
The hair is dyed uniformly and intensely.
Next, the locks of hair thus dyed are then subjected to a test of several repeated shampoo washes so as to evaluate the fastness (persistence) of the colouring obtained with respect to shampoo washing.
Shampoo wash protocol:
The locks are washed with standard shampoo (Garnier Ultra Doux) respectively at T=0 (i.e. immediately after the application of the dye composition to the keratin fibres).
The locks of hair are then rinsed, combed and dried with a hairdryer.
The next shampoo wash is then performed on the locks obtained after the application of the hairdryer.

Results:

The persistence of the colour of the locks was evaluated in the CIE L*a*b* system, using a Minolta Spectrophotometer CM3600D colorimeter (illuminant D65, angle 10°, specular component included).
In this L*a*b* system, L* represents the intensity of the colour, a* indicates the green/red colour axis and b* the blue/yellow colour axis.
The persistence of the colouring is evaluated by the colour difference ΔE between the dyed locks before shampooing, then after having undergone 1 and 5 shampoo washes according to the protocol described above. The lower the value of ΔE, the more persistent the colour with respect to shampoo washing.
The ΔE value is calculated according to the following equation:

[Math.1]

$$\Delta E = \sqrt{(L^* - L_o{}^*)^2 + (a^* - a_o{}^*)^2 + (b^* - b_o{}^*)^2}$$

In this equation, L*a*b* represent the values measured after dyeing the hair and after having undergone shampoo washes, and L0*a0*b0* represent the values measured after dyeing the hair but before shampoo washing.

[Tables 2]

| Compositions | Number of shampoo washes | L* | a* | b* | ΔE |
|---|---|---|---|---|---|
| Composition A | 0 | 34.1 | 30.39 | 20.2 | 0 |
| | 1 | 32.14 | 30.86 | 20.38 | 2.02 |
| | 5 | 37.19 | 27.08 | 18.43 | 4.86 |

(continued)

| Compositions | Number of shampoo washes | L* | a* | b* | ΔE |
|---|---|---|---|---|---|
| Composition B | 0 | 45.35 | 30.3 | 25.66 | 0 |
| | 1 | 42.22 | 31.21 | 26.72 | 3.43 |
| | 5 | 48.49 | 26.92 | 23.41 | 5.13 |

The locks of hair dyed with composition A according to the invention or composition B according to the invention and washed with one or five shampoo washes have low ΔE values.

Thus, the coloured coating of the keratin fibres using the compositions according to the invention shows good persistence with respect to shampoo washing. Specifically, the locks of hair dyed with the compositions according to the invention and washed with one or five shampoo washes show good persistence of the colour.

**Example 2:**

Solution 1 (i.e. solution of 3-aminopropyltriethoxysilane (APTES)) is prepared according to the process below:

- 15 g of APTES (APTES Sil soft A-1100 sold by the company Momentive Performance Materials) are mixed with 1.83 g of HCl (37% solution, Carlo Erba), and the solution is made up to 100 g with water. The solution is placed on a VWR brand magnetic stirrer (rotation speed 500 rpm) for 2 hours at room temperature.

The alcoholic solution 2 (i.e. alcoholic solution of non-amino silicone) is prepared according to the process below:

- the polydimethylsiloxane (PDMS) compound bearing hydroxyl end functions (PDMS reference 481939 sold by the company Sigma-Aldrich) is diluted in an ethanol solution in which the pigment (iron oxide sold by the company Sun Chemical under the name SunPuro Red Iron Oxide®) is dispersed.

Solution 1 is mixed with solution 2 and said mixture is applied to the keratin fibres.

The various compounds are present in the amounts below in g and also the mass ratio in the final APTES/PDMS mixture.

[Tables 3]

| Chemical name | | Composition C | Compositi on D | Composition E | Composition F | Composition G | Composition H |
|---|---|---|---|---|---|---|---|
| Solution 1 | | 18 | 13 | 10 | 7 | 2 | 10 |
| Solution 2 | Polydimethyl siloxanes (PDMS) bearing hydroxyl end functions | 0.3 | 1 | 1.5 | 2 | 2.7 | 1.5 |
| | Pigment (iron oxide sold by the company Sun Chemical under the name | 0.3 | 0.3 | 0.3 | 0.3 | 0.3 | - |
| | SunPuro Red Iron Oxide®) | | | | | | |
| | Pigment (D&C Red 7 sold by the company LCW Sensient under the name Unipure Red LC 3079) | - | - | - | - | - | 0.3 |
| | Ethanol | 7.35 | 7.70 | 7.95 | 8.20 | 8.55 | 7.95 |
| APTES/PDMS ratio | | 90/10 | 67/33 | 50/50 | 33/67 | 10/90 | 50/50 |

Protocol:

The cosmetic compositions (mixture of solution 1 and of solution 2 in the various ratios) are applied to locks of dry natural hair containing 90% white hairs, at a rate of 1 g of composition per gram of lock.

The locks of hair are then combed and dried with a hairdryer.

The hair is dyed uniformly and intensely.

Next, the locks of hair thus dyed are then subjected to a test of several repeated shampoo washes so as to evaluate the fastness (persistence) of the colouring obtained with respect to shampoo washing.

The shampoo wash protocol is identical to that described in Example 1.

Results:

The persistence of the colour of the locks was evaluated in the CIE L*a*b* system, using a Minolta Spectrophotometer CM3600D colorimeter (illuminant D65, angle 10°, specular component included), as explained in Example 1.

[Tables 4]

| Compositions | Number of shampoo washes | L* | a* | b* | ΔE |
|---|---|---|---|---|---|
| Composition C | 0 | 30.74 | 34.93 | 23.92 | 0 |
| | 1 | 27.09 | 32.43 | 20.11 | 5.84 |
| | 5 | 30.12 | 34.17 | 21.41 | 2.69 |
| Composition D | 0 | 32.06 | 33.08 | 22.32 | 0 |
| | 1 | 30.05 | 31.9 | 20.67 | 2.86 |
| | 5 | 31.12 | 32.6 | 20.82 | 1.83 |
| Composition E | 0 | 33.96 | 34.29 | 24.22 | 0 |
| | 1 | 32.11 | 34.15 | 23.22 | 2.11 |
| | 5 | 31.64 | 32.88 | 21.30 | 3.99 |
| Composition F | 0 | 27.85 | 34.26 | 23.09 | 0 |
| | 1 | 27.14 | 33.29 | 21.1 | 2.32 |
| | 5 | 29.9 | 32.57 | 20.6 | 3.64 |
| Composition G | 0 | 30.69 | 32.9 | 22.75 | 0 |
| | 1 | 30.42 | 34.88 | 23.62 | 2.18 |
| | 5 | 32.11 | 35.31 | 24.15 | 3.13 |
| Composition H | 0 | 27.36 | 41.51 | 59.1 | 0 |
| | 1 | 26.66 | 40.91 | 59.2 | 0.93 |
| | 5 | 26.65 | 39.88 | 58.4 | 1.91 |

The locks of hair dyed with compositions C to H according to the invention and washed with one or five shampoo washes have low ΔE values.

Thus, the coloured coating of the keratin fibres using the compositions according to the invention shows good persistence with respect to shampoo washing. Specifically, the locks of hair dyed with the compositions according to the invention and washed with one or five shampoo washes show good persistence of the colour.

**Example 3: Use of TEOS**

Solution 1 (i.e. solution of tetraethoxysilane (TEOS)) is prepared according to the process below:

- pure TEOS (reference 86578 sold by the company Sigma-Aldrich) is mixed with an aqueous solution of pH=1 by adding HCl, then an ethanol solution is added and said mixture is placed on a VWR brand magnetic stirrer (rotation speed 500 rpm) for 24 hours at room temperature.

The alcoholic solution 2 (i.e. alcoholic solution of non-amino silicone) is prepared according to the process below:

- the polydimethylsiloxane (PDMS) compound bearing hydroxyl end functions (reference 481939 sold by the company Sigma-Aldrich) is diluted in an ethanol solution in which the pigment (iron oxide sold by the company Sun Chemical under the name SunPuro Red Iron Oxide®) is dispersed.

Solution 1 is mixed with solution 2 and said mixture is applied to the keratin fibres.
The various compounds are present in the amounts below in g and also the mass ratio in the final TEOS/PDMS mixture.

[Tables 5]

| Chemical name | | Composition I | Composition J | Composition K | Composition L | Composition M |
|---|---|---|---|---|---|---|
| Solution 1 | Tetraethox ysilane (TEOS) | 2.7 | 2 | 1.5 | 1 | 0.3 |
| | Ethanol | 1.19 | 0.89 | 0.66 | 0.44 | 0.13 |
| | Water | 0.16 | 0.12 | 0.09 | 0.06 | 0.02 |
| Solution 2 | Polydimeth ylsiloxanes (PDMS) bearing hydroxyl end functions | 0.3 | 1 | 1.5 | 2 | 2.7 |
| | Pigment | 0.3 | 0.3 | 0.3 | 0.3 | 0.3 |
| | Ethanol | 7.35 | 7.70 | 7.95 | 8.20 | 8.55 |
| TEOS/PDMS ratio | | 90/10 | 67/33 | 50/50 | 33/67 | 10/90 |

Protocol:

The cosmetic compositions (mixture of solution 1 and of solution 2 in the various ratios) are applied to locks of dry natural hair containing 90% white hairs, at a rate of 1 g of composition per gram of lock.
The locks of hair are then combed and dried with a hairdryer.
The hair is dyed uniformly and intensely.
Next, the locks of hair thus dyed are then subjected to a test of several repeated shampoo washes so as to evaluate the fastness (persistence) of the colouring obtained with respect to shampoo washing.
The shampoo wash protocol is identical to that described in Example 1.

Results:

The persistence of the colour of the locks was evaluated in the CIE L*a*b* system, using a Minolta Spectrophotometer CM3600D colorimeter (illuminant D65, angle 10°, specular component included), as explained in Example 1.

[Tables 6]

| Compositions | Number of shampoo washes | L* | a* | b* | ΔE |
|---|---|---|---|---|---|
| Composition I | 0 | 34.58 | 30.32 | 22.48 | 0 |
| | 1 | 40.16 | 28.86 | 22.15 | 5.78 |
| | 5 | 41.02 | 26.27 | 20.21 | 7.94 |
| Composition J | 0 | 38.46 | 31.99 | 23.46 | 0 |
| | 1 | 37.69 | 32.61 | 25.15 | 1.96 |
| | 5 | 45.99 | 26.58 | 20.58 | 9.71 |

(continued)

| Compositions | Number of shampoo washes | L* | a* | b* | ΔE |
|---|---|---|---|---|---|
| Composition K | 0 | 35.53 | 34.78 | 26.1 | 0 |
| | 1 | 38.51 | 31.64 | 23.84 | 4.88 |
| | 5 | 42.14 | 25.88 | 19.95 | 12.68 |
| Composition L | 0 | 36.26 | 33.02 | 23.18 | 0 |
| | 1 | 35.35 | 33.1 | 24.73 | 1.80 |
| | 5 | 40.91 | 26.7 | 19.99 | 8.47 |
| Composition M | 0 | 37.1 | 30.75 | 21.61 | 0 |
| | 1 | 38.25 | 32.96 | 25.12 | 4.30 |
| | 5 | 40.6 | 26.52 | 20.99 | 5.53 |

The locks of hair dyed with compositions I to M according to the invention and washed with one or five shampoo washes have low ΔE values.

Thus, the coloured coating of the keratin fibres using the compositions according to the invention shows good persistence with respect to shampoo washing. Specifically, the locks of hair dyed with the compositions according to the invention and washed with one or five shampoo washes show good persistence of the colour.

**Example 4: Use of TEOS and APTES**

Solution 1 (i.e. solution of tetraethoxysilane (TEOS) and of 3-aminopropyltriethoxysilane (APTES)) is prepared according to the process below:

- pure TEOS (reference 86578 sold by the company Sigma-Aldrich) and APTES (APTES Silsoft A-1100 sold by the company Momentive Performance Materials) are mixed with an aqueous solution at pH=1 by adding HCl, then an ethanol solution is added and said mixture is placed on a VWR brand magnetic stirrer (rotation speed 500 rpm) for 24 hours at room temperature.

The alcoholic solution 2 (i.e. alcoholic solution of non-amino silicone) is prepared according to the process below:

- the polydimethylsiloxane (PDMS) compound bearing hydroxyl end functions (reference 481939 sold by the company Sigma-Aldrich) is diluted in an ethanol solution in which the pigment (iron oxide sold by the company Sun Chemical under the name SunPuro Red Iron Oxide®) is dispersed.

Solution 1 is mixed with solution 2 and said mixture is applied to the keratin fibres.

The various compounds are present in the amounts below in g and also the mass ratio in the final TEOS-APTES/PDMS mixture.

[Tables 7]

| Chemical name | | Composition N |
|---|---|---|
| Solution 1 | Tetraethoxysilane (TEOS) | 0.75 |
| | 3-Aminopropyltriethoxysilane (APTES) | 0.75 |
| | Ethanol | 0.66 |
| | Water | 0.09 |
| Solution 2 | Polydimethylsiloxanes (PDMS) bearing hydroxyl end functions | 1.5 |
| | Pigment | 0.3 |
| | Ethanol | 7.95 |
| TEOS-APTES/PDMS ratio | | 25+25/50 |

Protocol:

The cosmetic composition (mixture of solution 1 and of solution 2 in the various ratios) is applied to locks of dry natural hair containing 90% white hairs, at a rate of 1 g of composition per gram of lock.
The locks of hair are then combed and dried with a hairdryer.
The hair is dyed uniformly and intensely.
Next, the locks of hair thus dyed are then subjected to a test of several repeated shampoo washes so as to evaluate the fastness (persistence) of the colouring obtained with respect to shampoo washing.
The shampoo wash protocol is identical to that described in Example 1.

Results:

The persistence of the colour of the locks was evaluated in the CIE L*a*b* system, using a Minolta Spectrophotometer CM3600D colorimeter (illuminant D65, angle 10°, specular component included), as explained in Example 1.

[Tables 8]

| Compositions | Number of shampoo washes | L* | a* | b* | ΔE |
|---|---|---|---|---|---|
| Composition N | 0 | 33.31 | 32.87 | 21.4 | 0 |
| | 1 | 34.49 | 32.76 | 24.03 | 2.88 |
| | 5 | 37.09 | 31.45 | 22.96 | 4.33 |

The locks of hair dyed with composition N according to the invention and washed with one or five shampoo washes have low ΔE values.
Thus, the coloured coating of the keratin fibres using a composition according to the invention shows good persistence with respect to shampoo washing. Specifically, the locks of hair dyed with the composition according to the invention and washed with one or five shampoo washes show good persistence of the colour.

**Example 5: Use of TEOS and APTES**

Solution 1 (i.e. solution of 3-aminopropyltriethoxysilane (APTES)) is prepared according to the process below:

- 15 g of APTES (APTES Sil soft A-1100 sold by the company Momentive Performance Materials) are mixed with 1.83 g of HCl and the solution is made up to 100 g with water. The solution is placed on a VWR brand magnetic stirrer (rotation speed 500 rpm) for 2 hours at room temperature.

The alcoholic solution 2 (i.e. alcoholic solution of non-amino silicone) is prepared according to the process below:

- the polydimethylsiloxane (PDMS) compound bearing hydroxyl end functions (reference 481939 sold by the company Sigma-Aldrich) is diluted in an ethanol solution in which the pigment (iron oxide sold by the company Sun Chemical under the name SunPuro Red Iron Oxide®) and tetraethoxysilane (TEOS, 86578 sold by the company Sigma-Aldrich) are dispersed.

Solution 1 is mixed with solution 2 and said mixture is applied to the keratin fibres.

The various compounds are present in the amounts below in g and also the mass ratio in the final APTES-TEOS/PDMS mixture.

[Tables 9]

| Chemical name | | Composition O |
|---|---|---|
| Solution 1 | 3-Aminopropyltriethoxysilane (APTES) | 0.75 |
| | Ethanol | 0.66 |
| | Water | 0.09 |

(continued)

| Chemical name | | Composition O |
|---|---|---|
| Solution 2 | Polydimethylsiloxanes (PDMS) bearing hydroxyl end functions | 1.5 |
| | Tetraethoxysilane (TEOS) | 0.75 |
| | Pigment | 0.3 |
| | Ethanol | 7.95 |
| TEOS-APTES/PDMS ratio | | 25+25/50 |

Protocol:

The cosmetic composition (mixture of solution 1 and of solution 2 in the various ratios) is applied to locks of dry natural hair containing 90% white hairs, at a rate of 1 g of composition per gram of lock.

The locks of hair are then combed and dried with a hairdryer.

The hair is dyed uniformly and intensely.

Next, the locks of hair thus dyed are then subjected to a test of several repeated shampoo washes so as to evaluate the fastness (persistence) of the colouring obtained with respect to shampoo washing.

The shampoo wash protocol is identical to that described in Example 1.

Results:

The persistence of the colour of the locks was evaluated in the CIE L*a*b* system, using a Minolta Spectrophotometer CM3600D colorimeter (illuminant D65, angle 10°, specular component included), as explained in Example 1.

[Tables 10]

| Compositions | Number of shampoo washes | L* | a* | b* | ΔE |
|---|---|---|---|---|---|
| Composition O | 0 | 27.61 | 34.69 | 22.17 | 0 |
| | 1 | 28.46 | 32.41 | 20.8 | 2.79 |
| | 5 | 30.70 | 32.92 | 21.3 | 3.67 |

The locks of hair dyed with composition O according to the invention and washed with one or five shampoo washes have low ΔE values.

Thus, the coloured coating of the keratin fibres using a composition according to the invention shows good persistence with respect to shampoo washing. Specifically, the locks of hair dyed with the composition according to the invention and washed with one or five shampoo washes show good persistence of the colour.

**Example 6: Comparative example with non-hydrolysed APTES**

Comparative composition Q: A polydimethylsiloxane (PDMS) compound bearing hydroxyl end functions (reference 481939 sold by the company Sigma-Aldrich) is diluted in an ethanol solution in which the pigment (iron oxide sold by the company Sun Chemical under the name SunPuro Red Iron Oxide®) and APTES (APTES Silsoft A-1100 sold by the company Momentive Performance Materials) are dispersed.

The APTES is not hydrolysed, comparative composition Q does not comprise any water.

The various compounds are present in the amounts below in g and also the mass ratio in the final APTES/PDMS mixture.

[Tables 11]

| Chemical name | Composition Q (comparative) |
|---|---|
| Polydimethylsiloxanes (PDMS) bearing hydroxyl end functions | 1.5 |
| Pigment | 0.3 |
| 3-Aminopropyltriethoxysilane (APTES) | 1.5 |
| Ethanol | 7.95 |

(continued)

| Chemical name | Composition Q (comparative) |
|---|---|
| APTES/PDMS ratio | 50/50 |

Comparative composition Q is applied to locks of dry natural hair containing 90% white hairs, at a rate of 1 g of composition per gram of lock.

The locks of hair are then combed and dried with a hairdryer.

Next, the locks of hair thus dyed are then subjected to a test of several repeated shampoo washes so as to evaluate the fastness (persistence) of the colouring obtained with respect to shampoo washing.

The shampoo wash protocol is identical to that described in Example 1.

Results:

The persistence of the colour of the locks was evaluated in the CIE L*a*b* system, using a Minolta Spectrophotometer CM3600D colorimeter (illuminant D65, angle 10°, specular component included), as explained in Example 1.

[Tables 12]

| Compositions | Number of shampoo washes | L* | a* | b* | ΔE |
|---|---|---|---|---|---|
| Composition Q (comparative) | 0 | 30.85 | 35.63 | 24.12 | 0 |
| | 1 | 31.77 | 33.47 | 22.46 | 2.88 |
| | 5 | 36.14 | 30.75 | 22.58 | 7.36 |
| Composition E (invention) | 0 | 33.96 | 34.29 | 24.22 | 0 |
| | 1 | 32.11 | 34.15 | 23.22 | 2.11 |
| | 5 | 31.64 | 32.88 | 21.30 | 3.99 |

The locks of hair dyed with composition E according to the invention and washed with one or five shampoo washes have lower ΔE values than the locks of hair dyed with comparative composition Q.

Thus, the coloured coating of the keratin fibres using a composition according to the invention shows good persistence with respect to shampoo washing.

## Claims

**1.** Composition (C) for treating keratin fibres, notably human keratin fibres such as the hair, comprising:

a) at least one alkoxysilane chosen from the compounds of formula (I) below, oligomers thereof and/or mixtures thereof:

$$R^1_xSi(OR^2)_{(4-x)} \qquad (I)$$

in which:

- **$R^1$** represents an alkoxy group containing from 1 to 10 carbon atoms, a linear or branched, saturated or unsaturated, cyclic or acyclic $C_1$ to $C_{22}$, notably $C_1$ to $C_{20}$, hydrocarbon-based radical, which may be substituted with at least one group chosen from a hydroxyl group (OH); a thiol group; an amino group $NH_2$; an alkylamino group NHR in which R denotes a linear or branched alkyl radical containing from 1 to 20 carbon atoms, notably from 1 to 10 carbon atoms; an alkoxy group containing from 1 to 10 carbon atoms; a cycloalkyl containing from 3 to 40 carbon atoms; an aryl containing from 6 to 30 carbon atoms; $R^1$ possibly being interrupted with at least one heteroatom chosen from O, S, NH or a carbonyl group (CO);
- **$R^2$** represents a hydrogen atom or an alkyl group containing from 1 to 20 carbon atoms, preferably from 2 to 6 carbon atoms;
- **x** denotes an integer ranging from 1 to 3;

b) at least one non-amino silicone chosen from the compounds of formula (III) below:

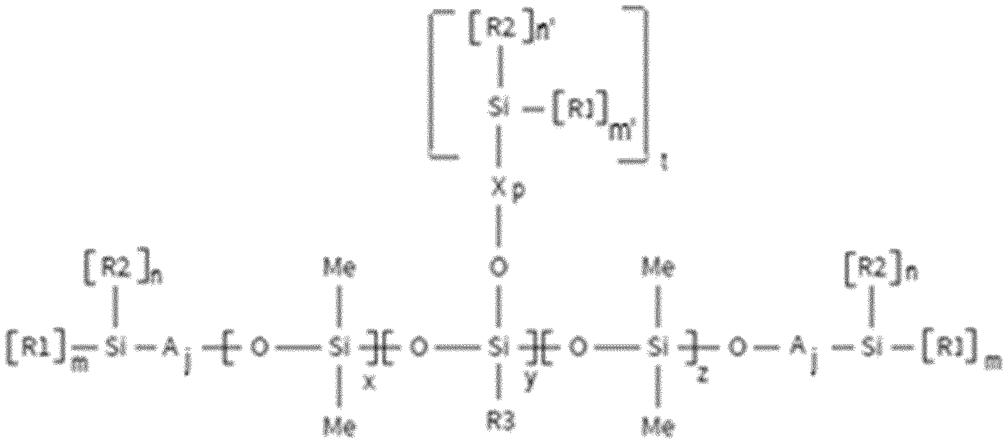

(III);

in which:

- **R1** independently represents a hydroxyl group or an alkoxy group containing from 1 to 2 carbon atoms;
- **R2** independently represents a hydrogen atom; an alkyl group containing from 1 to 20 carbon atoms, optionally substituted with at least one group chosen from a hydroxyl group (OH) or a thiol group, said alkyl group preferably being optionally substituted with at least one hydroxyl group (OH);
- **R3** represents a hydrogen atom; a hydroxyl group; an alkyl group containing from 1 to 10 carbon atoms, optionally substituted with at least one group chosen from a hydroxyl group (OH) or a thiol group; a cycloalkyl group containing from 3 to 20 carbon atoms, optionally substituted with at least one group chosen from a hydroxyl group (OH) or a thiol group; an alkoxy group containing from 1 to 2 carbon atoms, optionally substituted with at least one group chosen from a hydroxyl group (OH) or a thiol group; an aryl group containing from 6 to 12 carbon atoms, optionally substituted with at least one group chosen from a hydroxyl group (OH) or a thiol group;
- **A** represents an alkylene group containing from 1 to 2 carbon atoms;
- **X** represents a hydrogen atom, an alkyl group containing from 1 to 2 carbon atoms, a group -(R4-O)p- with **R4** representing a linear or branched alkylene group containing from 2 to 4 carbon atoms and **p** denoting an integer ranging from 0 to 3;
- **m** denotes an integer ranging from 1 to 3, **n** is an integer ranging from 0 to 2 and m+n=3;
- **m'** denotes an integer ranging from 1 to 3, **n'** is an integer ranging from 0 to 2 and m'+n'=3;
- **j** denotes an integer ranging from 0 to 1;
- **t** denotes an integer ranging from 0 to 1;
- **y** denotes an integer ranging from 0 to 10, z denotes an integer ranging from 0 to 500, with x+z ranging from 0 to 500 and $x+y+z \geq 4$; and

it being understood that if X represents a hydrogen atom, then t=0 and if p=0, then t=1;
c) water; and
d) at least one colouring agent chosen from pigments, direct dyes and mixtures thereof;

said composition being such that the alkoxysilane/non-amino silicone mass ratio ranges from 95/5 to 5/95.

**2.** Composition according to Claim 1, **characterized in that** the alkoxysilane(s) of formula (I), oligomers thereof and/or mixtures thereof are chosen from the compounds of formulae (Ia) and (Ib) below, alone or as a mixture:

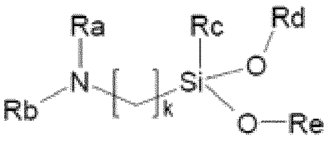 (Ia) 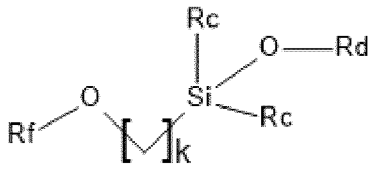

(Ib)

in which:

- **Ra** and **Rb**, which may be identical or different, represent a hydrogen atom; an alkyl group containing from 1 to 20 carbon atoms, preferably from 1 to 10 carbon atoms; a cycloalkyl group containing from 3 to 20 carbon atoms; an aryl group containing from 6 to 12 carbon atoms; an aminoalkyl group containing from 1 to 20 carbon atoms; a hydroxyalkyl group containing from 1 to 20 carbon atoms; an alkoxy group containing from 1 to 10 carbon atoms; an alkylcarbonyl group containing from 1 to 10 carbon atoms, preferably methylcarbonyl (i.e. acetyl);
- **Rc** represents a hydrogen atom; an alkyl group containing from 1 to 20 carbon atoms, preferably a methyl; an alkoxy group containing from 1 to 10 carbon atoms, preferably an ethoxy group; or an alkylaryl group containing from 7 to 12 carbon atoms;
- **Rd** and **Re**, which may be identical or different, represent an alkyl group containing from 1 to 20 carbon atoms, preferably an ethyl;
- **k** denotes an integer ranging from 0 to 20, preferably ranging from 0 to 3;
- **Rf** represents a hydrogen atom; an alkyl group containing from 1 to 20 carbon atoms such as an ethyl group; or a group of formula (II) below:

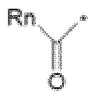

(II)

in which **Rn** represents a hydroxyl group (OH); an alkyl group containing from 1 to 20 carbon atoms, preferably a methyl.

3. Composition according to either one of the preceding claims, **characterized in that** the alkoxysilane(s) chosen from the compounds of formula (I), oligomers thereof and/or mixtures thereof, are chosen from 3-aminopropyltriethoxysilane (APTES), 3-aminopropylmethyldiethoxysilane (APMDES), N-cyclohexylaminomethyltriethoxysilane, tetraethoxysilane (TEOS), methyltriethoxysilane (MTES), dimethyldiethoxysilane (DMDES), diethyldiethoxysilane, dipropyldiethoxysilane, propyltriethoxysilane, isobutyltriethoxysilane, phenyltriethoxysilane, phenylmethyldiethoxysilane, diphenyldiethoxysilane, benzyltriethoxysilane, benzylmethyldiethoxysilane, dibenzyldiethoxysilane, acetoxymethyltriethoxysilane and mixtures thereof, preferably 3-aminopropyltriethoxysilane (APTES), tetraethoxysilane (TEOS) and mixtures thereof.

4. Composition according to any one of the preceding claims, **characterized in that** the alkoxysilane(s) of formula (I), oligomers thereof and/or mixtures thereof are present in a total amount ranging from 0.1% to 30% by weight, preferably from 0.5% to 25% by weight, better still from 0.5% to 20% by weight, relative to the total weight of the composition.

5. Composition according to any one of the preceding claims, **characterized in that** the compounds of formula (III) are such that:

- **R1** independently represents a hydroxyl group or an alkoxy group containing from 1 to 2 carbon atoms;
- **R2** independently represents a hydrogen atom or an alkyl group containing from 1 to 10 carbon atoms, preferably a methyl;
- **R3** represents a hydrogen atom; a hydroxyl group; an alkyl group containing from 1 to 10 carbon atoms; an alkoxy group containing from 1 to 2 carbon atoms or an aryl group containing from 6 to 12 carbon atoms, optionally substituted with a group chosen from a hydroxyl group (OH) or a thiol group;
- **X** represents a hydrogen atom or an alkyl group containing from 1 to 2 carbon atoms, and **p** denotes an integer ranging from 0 to 3;

- **m'** denotes an integer ranging from 1 to 3, **n'** denotes an integer ranging from 0 to 2 and m'+n'=3;
- **m** denotes an integer ranging from 1 to 3, **n** denotes an integer ranging from 0 to 2 and m+n=3;
- **j** is equal to 0;
- **t** denotes an integer ranging from 0 to 1; and
- **y** denotes an integer ranging from 0 to 10, z denotes an integer ranging from 0 to 500, with x+z ranging from 0 to 500 and x+y+z≥4; and

it being understood that if X represents a hydrogen atom, then t=0 and if p=0, then t=1.

**6.** Composition according to any one of the preceding claims, **characterized in that** the non-amino silicone(s) are chosen from the compounds of formula (IIIa) below:

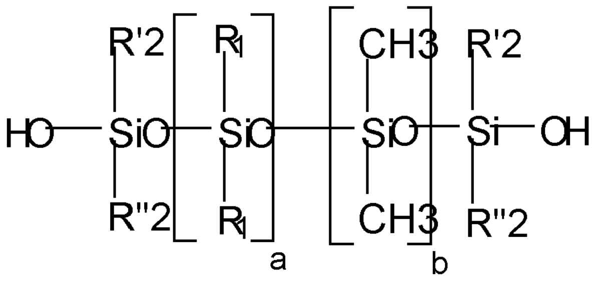

(IIIa)

in which:

- $\mathbf{R_1}$ independently represents a hydroxyl group; an alkyl group containing from 1 to 10 carbon atoms, preferably a methyl; an aryl group containing from 6 to 12 carbon atoms; an alkoxy group containing from 1 to 2 carbon atoms; or a group -O-Si(R'$_2$)$_3$;
- $\mathbf{R'_2}$ and $\mathbf{R''_2}$ independently represent a hydrogen atom; a hydroxyl group; or an alkyl group containing from 1 to 10 carbon atoms, preferably a methyl;
- **a** denotes an integer ranging from 0 to 10, **b** denotes an integer ranging from 0 to 500, with a+b≥4.

**7.** Composition according to Claim 6, **characterized in that** the non-amino silicone(s) of formula (IIIa) are such that:

- $\mathbf{R_1}$ independently represents an alkyl group containing from 1 to 10 carbon atoms, preferably a methyl;
- $\mathbf{R'_2}$ and $\mathbf{R''_2}$ independently represent a hydroxyl group or an alkyl group containing from 1 to 10 carbon atoms, preferably an alkyl group, more preferentially a methyl;
- **b** denotes an integer ranging from 0 to 10, **a** denotes an integer ranging from 0 to 5, with a+b≥4.

**8.** Composition according to any one of the preceding claims, **characterized in that** the non-amino silicone(s) of formula (III) are present in a total amount ranging from 0.1% to 30% by weight, preferably from 0.1% to 25% by weight, relative to the total weight of the composition.

**9.** Composition according to any one of the preceding claims, **characterized in that** the alkoxysilane/non-amino silicone mass ratio ranges from 90/10 to 10/90, preferentially from 60/40 to 40/60; even more preferentially, the alkoxysilane/non-amino silicone mass ratio is 50/50.

**10.** Composition according to any one of the preceding claims, **characterized in that** composition (C) comprises one or more organic solvents chosen from $C_1$-$C_4$ lower alkanols, more preferentially ethanol.

**11.** Use of the composition as defined in any one of Claims 1 to 10, for the cosmetic treatment, in particular dyeing, of keratin fibres, in particular human keratin fibres such as the hair.

**12.** Device for treating human keratin fibres such as the hair, comprising two compartments containing:

- in a first compartment (C1), a composition (A) comprising at least one alkoxysilane chosen from the compounds of formula (I), oligomers thereof and/or mixtures thereof as defined in any one of Claims 1 to 4;
- in a second compartment (C2), a composition (B) comprising at least one non-amino silicone chosen from the

compounds of formula (III) as defined in any one of Claims 1 and 5 to 8; and at least one colouring agent chosen from pigments, direct dyes and mixtures thereof;

it being understood that composition (A) and/or composition (B) comprise water, and the mixing of the compositions of compartments (C1) and (C2) gives an alkoxysilane/non-amino silicone mass ratio ranging from 95/5 to 5/95.

**13.** Process for treating human keratin fibres such as the hair, which consists in extemporaneously mixing, at the time of use, two compositions (A) and (B) and in applying the mixture to the fibres, said mixture comprising an alkoxysilane/non-amino silicone mass ratio ranging from 95/5 to 5/95, with:

- composition (A) comprising at least one alkoxysilane chosen from the compounds of formula (I), oligomers thereof and/or mixtures thereof as defined in any one of Claims 1 to 4;
- composition (B) comprising at least one non-amino silicone chosen from the compounds of formula (III) as defined in any one of Claims 1 and 5 to 8; and at least one colouring agent chosen from pigments, direct dyes and mixtures thereof;

it being understood that composition (A) and/or composition (B) comprise water.

**14.** Process according to Claim 13, **characterized in that** the alkoxysilane/non-amino silicone mass ratio ranges from 90/10 to 10/90, preferably from 60/40 to 40/60; more preferentially, the alkoxysilane/non-amino silicone mass ratio is 50/50.

**15.** Process for dyeing keratin fibres, in particular human keratin fibres, preferably the hair, comprising:

- the application to said fibres of a composition comprising:

a) at least one alkoxysilane chosen from the compounds of formula (I), oligomers thereof and/or mixtures thereof as defined in any one of Claims 1 to 4;
b) at least one non-amino silicone chosen from the compounds of formula (III) as defined in any one of Claims 1 and 5 to 8;
c) water and
d) at least one colouring agent chosen from pigments, direct dyes and mixtures thereof.

**16.** Dyeing process according to Claim 15, **characterized in that** the alkoxysilane/non-amino silicone mass ratio ranges from 95/5 to 5/95, preferably from 90/10 to 10/90, more preferentially from 60/40 to 40/60; even more preferentially, the alkoxysilane/non-amino silicone mass ratio is 50/50.

## Patentansprüche

**1.** Zusammensetzung (C) zur Behandlung von Keratinfasern, insbesondere menschlichen Keratinfasern, wie z. B. dem Haar, umfassend:

a) wenigstens ein Alkoxysilan ausgewählt aus den Verbindungen der nachstehenden Formel (I), Oligomeren davon und/oder Gemischen davon:

$$R^1{}_xSi(OR^2)_{(4-x)} \qquad (I)$$

wobei:

- **$R^1$** eine Alkoxygruppe mit von 1 bis 10 Kohlenstoffatomen, einen linearen oder verzweigten, gesättigten oder ungesättigten, cyclischen oder acyclischen $C_1$- bis $C_{22}$-, insbesondere $C_1$- bis $C_{20}$-, Rest auf Kohlenwasserstoffbasis darstellt, der substituiert sein kann mit wenigstens einer Gruppe ausgewählt aus einer Hydroxygruppe (OH); einer Thiolgruppe; einer Aminogruppe $NH_2$; einer Alkylaminogruppe NHR, wobei R einen linearen oder verzweigten Alkylrest mit von 1 bis 20 Kohlenstoffatomen, insbesondere von 1 bis 10 Kohlenstoffatomen, darstellt; einer Alkoxygruppe mit von 1 bis 10 Kohlenstoffatomen; einem Cycloalkyl mit von 3 bis 40 Kohlenstoffatomen; einem Aryl mit von 6 bis 30 Kohlenstoffatomen; wobei $R^1$ gegebenenfalls von wenigstens einem Heteroatom ausgewählt aus O, S, NH oder einer Carbonylgruppe (CO) unterbrochen

ist;
- $R^2$ ein Wasserstoffatom oder eine Alkylgruppe mit von 1 bis 20 Kohlenstoffatomen, vorzugsweise von 2 bis 6 Kohlenstoffatomen, darstellt;
- **x** eine ganze Zahl in dem Bereich von 1 bis 3 bezeichnet;

b) wenigstens ein Nichtamino-Silicon ausgewählt aus den Verbindungen der nachstehenden Formel (III):

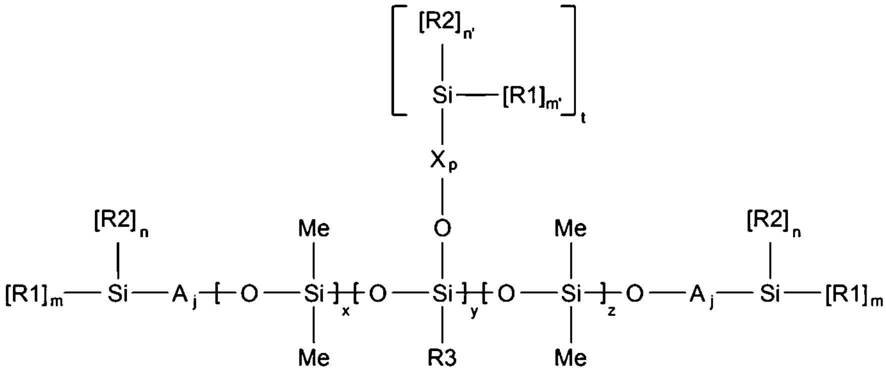

(III)

wobei:

- $R^1$ unabhängig eine Hydroxygruppe oder eine Alkoxygruppe mit von 1 bis 2 Kohlenstoffatomen darstellt;
- $R^2$ unabhängig ein Wasserstoffatom; eine Alkylgruppe mit von 1 bis 20 Kohlenstoffatomen, gegebenenfalls substituiert mit wenigstens einer Gruppe ausgewählt aus einer Hydroxygruppe (OH) und einer Thiolgruppe, wobei die Alkylgruppe vorzugsweise gegebenenfalls mit wenigstens einer Hydroxygruppe (OH) substituiert ist, darstellt;
- $R^3$ ein Wasserstoffatom; eine Hydroxygruppe; eine Alkylgruppe mit von 1 bis 10 Kohlenstoffatomen, gegebenenfalls substituiert mit wenigstens einer Gruppe ausgewählt aus einer Hydroxygruppe (OH) und einer Thiolgruppe; eine Cycloalkylgruppe mit von 3 bis 20 Kohlenstoffatomen, gegebenenfalls substituiert mit wenigstens einer Gruppe ausgewählt aus einer Hydroxygruppe (OH) und einer Thiolgruppe; eine Alkoxygruppe mit von 1 bis 2 Kohlenstoffatomen, gegebenenfalls substituiert mit wenigstens einer Gruppe ausgewählt aus einer Hydroxygruppe (OH) und einer Thiolgruppe; eine Arylgruppe mit von 6 bis 12 Kohlenstoffatomen, gegebenenfalls substituiert mit wenigstens einer Gruppe ausgewählt aus einer Hydroxygruppe (OH) und einer Thiolgruppe, darstellt;
- **A** eine Alkylengruppe mit von 1 bis 2 Kohlenstoffatomen darstellt;
- **X** ein Wasserstoffatom, eine Alkylgruppe mit von 1 bis 2 Kohlenstoffatomen, eine Gruppe -(R4-O)p-, wobei **R4** eine lineare oder verzweigte Alkylengruppe mit von 2 bis 4 Kohlenstoffatomen darstellt und **p** eine ganze Zahl in dem Bereich von 0 bis 3 bezeichnet, darstellt;
- **m** eine ganze Zahl in dem Bereich von 1 bis 3 bezeichnet, **n** eine ganze Zahl in dem Bereich von 0 bis 2 ist und m+n=3;
- **m'** eine ganze Zahl in dem Bereich von 1 bis 3 bezeichnet, **n'** eine ganze Zahl in dem Bereich von 0 bis 2 ist und m'+n'=3;
- **j** eine ganze Zahl in dem Bereich von 0 bis 1 bezeichnet;
- **t** eine ganze Zahl in dem Bereich von 0 bis 1 bezeichnet;
- **y** eine ganze Zahl in dem Bereich von 0 bis 10 bezeichnet, **z** eine ganze Zahl in dem Bereich von 0 bis 500 bezeichnet, wobei x+z in dem Bereich von 0 bis 500 liegt und $x+y+z \geq 4$; und

wobei zu beachten ist, dass, wenn X ein Wasserstoffatom darstellt, dann t=0, und, wenn p=0, dann t=1;
c) Wasser; und
d) wenigstens ein Farbmittel ausgewählt aus Pigmenten, Direktfarbstoffen und Gemischen davon;

wobei die Zusammensetzung so ist, dass das Masseverhältnis Alkoxysilan/Nichtamino-Silicon in dem Bereich von 95/5 bis 5/95 liegt.

**2.** Zusammensetzung gemäß Anspruch 1, **dadurch gekennzeichnet, dass** das/die Alkoxysilan(e) der Formel (I),

Oligomere davon und/oder Gemische davon ausgewählt sind aus den Verbindungen der nachstehenden Formeln (Ia) und (Ib), allein oder als ein Gemisch:

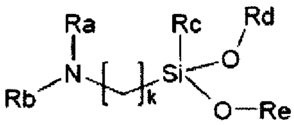 (Ia)

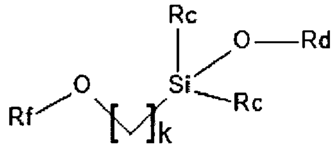 (Ib) ,

wobei:

- **Ra** und **Rb,** die gleich oder verschieden sein können, ein Wasserstoffatom; eine Alkylgruppe mit von 1 bis 20 Kohlenstoffatomen, vorzugsweise von 1 bis 10 Kohlenstoffatomen; eine Cycloalkylgruppe mit von 3 bis 20 Kohlenstoffatomen; eine Arylgruppe mit von 6 bis 12 Kohlenstoffatomen; eine Aminoalkylgruppe mit von 1 bis 20 Kohlenstoffatomen; eine Hydroxyalkylgruppe mit von 1 bis 20 Kohlenstoffatomen; eine Alkoxygruppe mit von 1 bis 10 Kohlenstoffatomen; eine Alkylcarbonylgruppe mit von 1 bis 10 Kohlenstoffatomen, vorzugsweise Methylcarbonyl (d. h. Acetyl), darstellen;
- **Rc** ein Wasserstoffatom; eine Alkylgruppe mit von 1 bis 20 Kohlenstoffatomen, vorzugsweise ein Methyl; eine Alkoxygruppe mit von 1 bis 10 Kohlenstoffatomen, vorzugsweise eine Ethoxygruppe; oder eine Alkylarylgruppe mit von 7 bis 12 Kohlenstoffatomen darstellt;
- **Rd** und **Re,** die gleich oder verschieden sein können, eine Alkylgruppe mit von 1 bis 20 Kohlenstoffatomen, vorzugsweise ein Ethyl, darstellen;
- **k** eine ganze Zahl in dem Bereich von 0 bis 20, vorzugsweise in dem Bereich von 0 bis 3, bezeichnet;
- **Rf** ein Wasserstoffatom; eine Alkylgruppe mit von 1 bis 20 Kohlenstoffatomen, wie z. B. eine Ethylgruppe; oder eine Gruppe der nachstehenden Formel (II) darstellt:

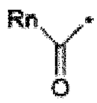 (II) ,

wobei **Rn** eine Hydroxygruppe (OH); eine Alkylgruppe mit von 1 bis 20 Kohlenstoffatomen, vorzugsweise ein Methyl, darstellt.

3. Zusammensetzung gemäß einem der vorstehenden Ansprüche, **dadurch gekennzeichnet, dass** das/die Alkoxysilan(e) ausgewählt aus den Verbindungen der Formel (I), Oligomeren davon und/oder Gemischen davon ausgewählt sind aus 3-Aminopropyltriethoxysilan (APTES), 3-Aminopropylmethyldiethoxysilan (APMDES), N-Cyclohexylaminomethyltriethoxysilan, Tetraethoxysilan (TEOS), Methyltriethoxysilan (MTES), Dimethyldiethoxysilan (DMDES), Diethyldiethoxysilan, Dipropyldiethoxysilan, Propyltriethoxysilan, Isobutyltriethoxysilan, Phenyltriethoxysilan, Phenylmethyldiethoxysilan, Diphenyldiethoxysilan, Benzyltriethoxysilan, Benzylmethyldiethoxysilan, Dibenzyldiethoxysilan, Acetoxymethyltriethoxysilan und Gemischen davon, vorzugsweise 3-Aminopropyltriethoxysilan (APTES), Tetraethoxysilan (TEOS) und Gemischen davon.

4. Zusammensetzung gemäß einem der vorstehenden Ansprüche, **dadurch gekennzeichnet, dass** das/die Alkoxysilan(e) der Formel (I), Oligomere davon und/oder Gemische davon in einer Gesamtmenge in dem Bereich von 0,1 Gew.-% bis 30 Gew.-%, vorzugsweise von 0,5 Gew.-% bis 25 Gew.-%, noch besser von 0,5 Gew.-% bis 20 Gew.-%, bezogen auf das Gesamtgewicht der Zusammensetzung, vorhanden sind.

5. Zusammensetzung gemäß einem der vorstehenden Ansprüche, **dadurch gekennzeichnet, dass** die Verbindungen der Formel (III) so sind, dass:

- **R1** unabhängig eine Hydroxygruppe oder eine Alkoxygruppe mit von 1 bis 2 Kohlenstoffatomen darstellt;
- **R2** unabhängig ein Wasserstoffatom oder eine Alkylgruppe mit von 1 bis 10 Kohlenstoffatomen, vorzugsweise ein Methyl, darstellt;
- **R3** ein Wasserstoffatom; eine Hydroxygruppe; eine Alkylgruppe mit von 1 bis 10 Kohlenstoffatomen; eine

Alkoxygruppe mit von 1 bis 2 Kohlenstoffatomen oder eine Arylgruppe mit von 6 bis 12 Kohlenstoffatomen, gegebenenfalls substituiert mit einer Gruppe ausgewählt aus einer Hydroxygruppe (OH) und einer Thiolgruppe, darstellt;
- **X** ein Wasserstoffatom oder eine Alkylgruppe mit von 1 bis 2 Kohlenstoffatomen darstellt und **p** eine ganze Zahl in dem Bereich von 0 bis 3 bezeichnet;
- **m'** eine ganze Zahl in dem Bereich von 1 bis 3 bezeichnet, **n'** eine ganze Zahl in dem Bereich von 0 bis 2 bezeichnet und m'+n'=3;
- **m** eine ganze Zahl in dem Bereich von 1 bis 3 bezeichnet, **n** eine ganze Zahl in dem Bereich von 0 bis 2 bezeichnet und m+n=3;
- **j** gleich 0 ist;
- **t** eine ganze Zahl in dem Bereich von 0 bis 1 bezeichnet; und
- **y** eine ganze Zahl in dem Bereich von 0 bis 10 bezeichnet, **z** eine ganze Zahl in dem Bereich von 0 bis 500 bezeichnet, wobei x+z in dem Bereich von 0 bis 500 liegt und x+y+z≥4; und

wobei zu beachten ist, dass, wenn X ein Wasserstoffatom darstellt, dann t=0, und, wenn p=0, dann t=1.

**6.** Zusammensetzung gemäß einem der vorstehenden Ansprüche, **dadurch gekennzeichnet, dass** das/die Nichtamino-Silicon(e) ausgewählt sind aus den Verbindungen der nachstehenden Formel (IIIa):

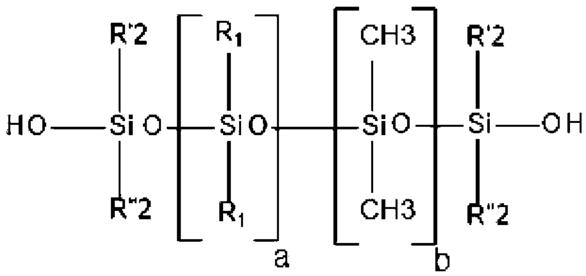

(IIIa)

wobei:

- $R^1$ unabhängig eine Hydroxygruppe; eine Alkylgruppe mit von 1 bis 10 Kohlenstoffatomen, vorzugsweise ein Methyl; eine Arylgruppe mit von 6 bis 12 Kohlenstoffatomen; eine Alkoxygruppe mit von 1 bis 2 Kohlenstoffatomen; oder eine Gruppe $-O-Si(R'_2)_3$ darstellt;
- $R'_2$ und $R''_2$ unabhängig ein Wasserstoffatom; eine Hydroxygruppe; oder eine Alkylgruppe mit von 1 bis 10 Kohlenstoffatomen, vorzugsweise ein Methyl, darstellen;
- **a** eine ganze Zahl in dem Bereich von 0 bis 10 bezeichnet, **b** eine ganze Zahl in dem Bereich von 0 bis 500 bezeichnet, wobei a+b≥4.

**7.** Zusammensetzung gemäß Anspruch 6, **dadurch gekennzeichnet, dass** das/die Nichtamino-Silicon(e) der Formel (IIIa) so sind, dass:

- $R^1$ unabhängig eine Alkylgruppe mit von 1 bis 10 Kohlenstoffatomen, vorzugsweise ein Methyl, darstellt;
- $R''_2$ und $R''_2$ unabhängig eine Hydroxygruppe oder eine Alkylgruppe mit von 1 bis 10 Kohlenstoffatomen, vorzugsweise eine Alkylgruppe, bevorzugter ein Methyl, darstellen;
- **b** eine ganze Zahl in dem Bereich von 0 bis 10 bezeichnet, **a** eine ganze Zahl in dem Bereich von 0 bis 5 bezeichnet, wobei a+b≥4.

**8.** Zusammensetzung gemäß einem der vorstehenden Ansprüche, **dadurch gekennzeichnet, dass** das/die Nichtamino-Silicon(e) der Formel (III) in einer Gesamtmenge in dem Bereich von 0,1 Gew.-% bis 30 Gew.-%, vorzugsweise von 0,1 Gew.-% bis 25 Gew.-%, bezogen auf das Gesamtgewicht der Zusammensetzung, vorhanden sind.

**9.** Zusammensetzung gemäß einem der vorstehenden Ansprüche, **dadurch gekennzeichnet, dass** das Masseverhältnis Alkoxysilan/Nichtamino-Silicon in dem Bereich von 90/10 bis 10/90, vorzugsweise von 60/40 bis 40/60, liegt; noch bevorzugter beträgt das Masseverhältnis Alkoxysilan/Nichtamino-Silicon 50/50.

**10.** Zusammensetzung gemäß einem der vorstehenden Ansprüche, **dadurch gekennzeichnet, dass** Zusammensetzung (C) ein oder mehrere organische Lösungsmittel ausgewählt aus $C_1$-$C_4$-Niederalkanolen, bevorzugter Ethanol, umfasst.

**11.** Verwendung der Zusammensetzung gemäß einem der Ansprüche 1 bis 10 für die kosmetische Behandlung, insbesondere Färbung, von Keratinfasern, insbesondere menschlichen Keratinfasern, wie z. B. dem Haar.

**12.** Vorrichtung zur Behandlung von menschlichen Keratinfasern, wie z. B. dem Haar, umfassend zwei Kammern, die enthalten:

- in einer ersten Kammer (C1) eine Zusammensetzung (A) umfassend wenigstens ein Alkoxysilan ausgewählt aus den Verbindungen der Formel (I), Oligomeren davon und/oder Gemischen davon gemäß einem der Ansprüche 1 bis 4;
- in einer zweiten Kammer (C2) eine Zusammensetzung (B) umfassend wenigstens ein Nichtamino-Silicon ausgewählt aus den Verbindungen der Formel (III) gemäß einem der Ansprüche 1 und 5 bis 8; und wenigstens ein Farbmittel ausgewählt aus Pigmenten, Direktfarbstoffen und Gemischen davon;

wobei zu beachten ist, dass Zusammensetzung (A) und/oder Zusammensetzung (B) Wasser umfassen und das Mischen der Zusammensetzungen der Kammern (C1) und (C2) ein Masseverhältnis Alkoxysilan/Nichtamino-Silicon in dem Bereich von 95/5 bis 5/95 ergibt.

**13.** Verfahren zur Behandlung von menschlichen Keratinfasern, wie z. B. dem Haar, bestehend aus unvorbereitetem Mischen zur Zeit der Verwendung von zwei Zusammensetzungen (A) und (B) und Auftragen des Gemischs auf die Fasern, wobei das Gemisch ein Masseverhältnis Alkoxysilan/Nichtamino-Silicon in dem Bereich von 95/5 bis 5/95 umfasst, wobei:

- Zusammensetzung (A) wenigstens ein Alkoxysilan ausgewählt aus den Verbindungen der Formel (I), Oligomeren davon und/oder Gemischen davon gemäß einem der Ansprüche 1 bis 4 umfasst;
- Zusammensetzung (B) wenigstens ein Nichtamino-Silicon ausgewählt aus den Verbindungen der Formel (III) gemäß einem der Ansprüche 1 und 5 bis 8; und wenigstens ein Farbmittel ausgewählt aus Pigmenten, Direktfarbstoffen und Gemischen davon umfasst;

wobei zu beachten ist, dass Zusammensetzung (A) und/oder Zusammensetzung (B) Wasser umfassen.

**14.** Verfahren gemäß Anspruch 13, **dadurch gekennzeichnet, dass** das Masseverhältnis Alkoxysilan/Nichtamino-Silicon in dem Bereich von 90/10 bis 10/90, vorzugsweise von 60/40 bis 40/60, liegt; bevorzugter beträgt das Masseverhältnis Alkoxysilan/Nichtamino-Silicon 50/50.

**15.** Verfahren zum Färben von Keratinfasern, insbesondere menschlichen Keratinfasern, vorzugsweise des Haars, umfassend:

- auf die Fasern Auftragen einer Zusammensetzung umfassend:

  a) wenigstens ein Alkoxysilan ausgewählt aus den Verbindungen der Formel (I), Oligomeren davon und/oder Gemischen davon gemäß einem der Ansprüche 1 bis 4;
  b) wenigstens ein Nichtamino-Silicon ausgewählt aus den Verbindungen der Formel (III) gemäß einem der Ansprüche 1 und 5 bis 8;
  c) Wasser und
  d) wenigstens ein Farbmittel ausgewählt aus Pigmenten, Direktfarbstoffen und Gemischen davon.

**16.** Färbeverfahren gemäß Anspruch 15, **dadurch gekennzeichnet, dass** das Masseverhältnis Alkoxysilan/Nichtamino-Silicon in dem Bereich von 95/5 bis 5/95, vorzugsweise von 90/10 bis 10/90, bevorzugter von 60/40 bis 40/60, liegt; sogar bevorzugter beträgt das Masseverhältnis Alkoxysilan/Nichtamino-Silicon 50/50.

## Revendications

**1.** Composition (C) de traitement des fibres kératiniques, notamment des fibres kératiniques humaines telles que les cheveux comprenant :

a) au moins un alcoxysilane choisi parmi les composés de formule (I) suivante, leurs oligomères et/ou leurs mélanges :

$$R^I_xSi(OR^2)_{(4-x)} \qquad (I)$$

dans laquelle :

- **R¹** représente un groupe alcoxy ayant de 1 à 10 atomes de carbone, un radical hydrocarboné en Ci à $C_{22}$, notamment en $C_1$ à $C_{20}$, linéaire ou ramifié, saturé ou insaturé, cyclique ou acyclique, pouvant être substitué par au moins un groupement choisi parmi un groupement hydroxy (OH) ; un groupement thiol ; un groupement amino $NH_2$ ; un groupement alkylamino NHR dans lequel R désigne un radical alkyle linéaire ou ramifié ayant de 1 à 20 atomes de carbone, notamment de 1 à 10 atomes de carbone ; un groupe alcoxy ayant de 1 à 10 atomes de carbone ; un cycloalkyle ayant de 3 à 40 atomes de carbone ; un aryle ayant de 6 à 30 atomes de carbone; $R^1$ pouvant être interrompu par au moins un hétéroatome choisi parmi O, S, NH ou un groupement carbonyle (CO) ;
- **R²** représente un atome d'hydrogène ou un groupe alkyle ayant de 1 à 20 atomes de carbone, de préférence de 2 à 6 atomes de carbone ;
- **x** désigne un nombre entier allant de 1 à 3 ;

b) au moins une silicone non aminée choisie parmi les composés de formule (III) suivante :

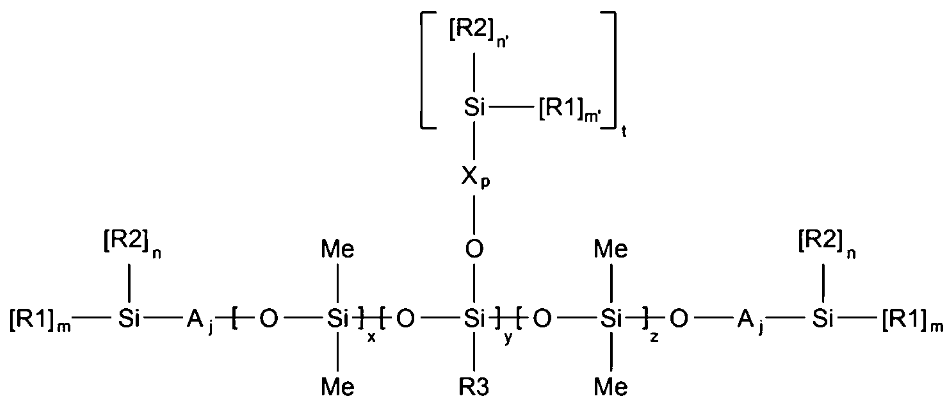

(III);

dans laquelle :

- **R1** représente indépendamment un groupement hydroxy ou un groupe alcoxy ayant de 1 à 2 atomes de carbone ;
- **R2** représente indépendamment un atome d'hydrogène ; un groupe alkyle ayant de 1 à 20 atomes de carbone éventuellement substitué par au moins un groupement choisi parmi un groupement hydroxy (OH) ou un groupement thiol , ledit groupe alkyle étant de préférence éventuellement substitué par au moins un groupement hydroxy (OH);
- **R3** représente un atome d'hydrogène ; un groupement hydroxy ; un groupe alkyle ayant de 1 à 10 atomes de carbone éventuellement substitué par au moins un groupement choisi parmi un groupement hydroxy (OH) ou un groupement thiol ; un groupe cycloalkyle ayant de 3 à 20 atomes de carbone éventuellement substitué par au moins un groupement choisi parmi un groupement hydroxy (OH) ou un groupement thiol ; un groupe alcoxy ayant de 1 à 2 atomes de carbone éventuellement substitué par au moins un groupement choisi parmi un groupement hydroxy (OH) ou un groupement thiol ; un groupe aryle ayant de 6 à 12 atomes de carbone éventuellement substitué par au moins un groupement choisi parmi un groupement hydroxy (OH) ou un groupement thiol ;
- **A** représente un groupe alkylène ayant de 1 à 2 atomes de carbone ;
- **X** représente un atome d'hydrogène, un groupe alkyle ayant de 1 à 2 atomes de carbone, un groupe -(R4-O)p- avec **R4** représentant un groupe alkylène, linéaire ou ramifié, ayant de 2 à 4 atomes de carbone et **p** désignant un nombre entier allant de 0 à 3 ;
- **m** désigne un nombre entier allant de 1 à 3, **n** est un nombre entier allant de 0 à 2 et m+n= 3 ;

- **m’** désigne un nombre entier allant de 1 à 3, **n’** est un nombre entier allant de 0 à 2 et m’+n’=3 ;
- **j** désigne un nombre entier allant de 0 à 1 ;
- **t** désigne un nombre entier allant de 0 à 1 ;
- y désigne un nombre entier allant de 0 à 10, z désigne un nombre entier allant de 0 à 500 avec x+z variant de 0 à 500 et x+y+z ≥4, et

étant entendu que si X représente un atome d’hydrogène, alors t=0 et si p=0, alors t=1 ;
c) de l’eau ; et
d) au moins un agent colorant choisi parmi les pigments, les colorants directs et leurs mélanges ;

ladite composition étant telle que le ratio massique d’ alcoxysilane/silicone non aminée varie de 95 :5 à 5 :95.

**2.** Composition selon la revendication 1, **caractérisée en ce que** le ou les alcoxysilanes de formule (I), leurs oligomères et/ou leurs mélanges sont choisis parmi les composés de formule (Ia) et (Ib) suivantes, seul ou en mélange :

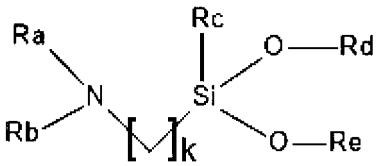
(Ia)

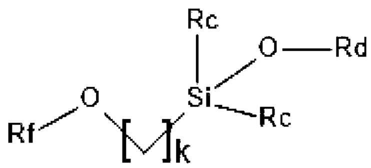
(Ib)

dans lesquelles :

- **Ra et Rb,** identiques ou différents, représentent un atome d’hydrogène ; un groupe alkyle ayant de 1 à 20 atomes de carbone, de préférence de 1 à 10 atomes de carbone ; un groupe cycloalkyle ayant de 3 à 20 atomes de carbone ; un groupe aryle ayant de 6 à 12 atomes de carbone ; un groupe aminoalkyle ayant de 1 à 20 atomes de carbone ; un groupe hydroxyalkyle ayant de 1 à 20 atomes de carbone ; un groupe alcoxy ayant de 1 à 10 atomes de carbone ; un groupe alkylcarbonyl ayant de 1 à 10 atomes de carbone, de préférence méthylcarbonyl (i.e. acétyl) ;
- **Rc** représente un atome d’hydrogène ; un groupe alkyle ayant de 1 à 20 atomes de carbone, de préférence un méthyl ; un groupe alcoxy ayant de 1 à 10 atomes de carbone, de préférence un groupe éthoxy ; ou un groupe alkylaryl ayant de 7 à 12 atomes de carbone ;
- **Rd et Re,** identiques ou différents, représentent un groupe alkyle ayant de 1 à 20 atomes de carbone, de préférence un éthyle ;
- **k** désigne un nombre entier allant de 0 à 20, de préférence allant de 0 à 3 ;
- **Rf** représente un atome d’hydrogène ; un groupe alkyle ayant de 1 à 20 atomes de carbone tel qu’un groupe éthyle ; ou un groupe de formule (II) suivante :

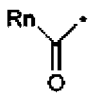
(II)

dans laquelle **Rn** représente un groupement hydroxy (OH) ; un groupe alkyle ayant de 1 à 20 atomes de carbone, de préférence un méthyl.

**3.** Composition selon l’une quelconque des revendication précédentes, **caractérisée en ce que** le ou les alcoxysilanes choisis parmi les composés de formule (I), leurs oligomères et/ou leurs mélanges, sont choisis parmi le 3-aminopropyltriethoxysilane (APTES), le 3-aminopropylmethyldiethoxysilane (APMDES), le N-cyclohexylaminomethyl triethoxysilane. le tetraethoxysilane (TEOS), le méthyltriéthoxysilane (MTES), le diméthyldiéthoxysilane (DMDES), le diéthyldiéthoxysilane, le dipropyldiéthoxysilane, le propyltriéthoxysilane, l’isobutyltriéthoxysilane, le phenyltriéthoxysilane, le phenylmethyldiéthoxysilane, le diphenyldiéthoxysilane, le benzyltriethoxysilane, le benzylmethyldiéthoxysilane, le dibenzyldiéthoxysilane, l’acétoxymethyltriethoxysilane et leurs mélanges, de préférence le 3-aminopropyltriethoxysilane (APTES), le tetraethoxysilane (TEOS) et leurs mélanges.

**4.** Composition selon l'une quelconque des revendications précédentes, **caractérisée en ce que** le ou les alcoxysilanes de formule (I), leurs oligomères et/ou leurs mélanges sont présents en une quantité totale allant de 0,1 à 30% en poids, de préférence de 0,5 à 25% en poids, encore mieux de 0,5 à 20% en poids, par rapport au poids total de la composition.

**5.** Composition selon l'une quelconque des revendication précédentes, **caractérisée en ce que** les composés de formule (III) sont tels que :

- **R1** représente indépendamment un groupement hydroxy ou un groupe alcoxy ayant de 1 à 2 atomes de carbone ;
- **R2** représente indépendamment un atome d'hydrogène ou un groupe alkyle ayant de 1 à 10 atomes de carbone, de préférence un méthyl ;
- **R3** représente un atome d'hydrogène ; un groupement hydroxy ; un groupe alkyle ayant de 1 à 10 atomes de carbone ; un groupe alcoxy ayant de 1 à 2 atomes de carbone ou un groupe aryle ayant de 6 à 12 atomes de carbone éventuellement substitué par un groupement choisi parmi un groupement hydroxy (OH) ou un groupement thiol ,
- **X** représente un atome d'hydrogène ou un groupe alkyle ayant de 1 à 2 atomes de carbone, et **p** désigne un nombre entier allant de 0 à 3,
- **m'** désigne un nombre entier allant de 1 à 3, **n'** désigne un nombre entier allant de 0 à 2 et m'+n'=3
- **m** désigne un nombre entier allant de 1 à 3, **n** désigne un nombre entier allant de 0 à 2 et m+n= 3 ;
- **j** est égal à 0 ;
- **t** désigne un nombre entier allant de 0 à 1 ;et
- **y** désigne un nombre entier allant de 0 à 10, z désigne un nombre entier allant de 0 à 500 avec x+z variant de 0 à 500 et x+y+z ≥4, et

étant entendu que si X représente un atome d'hydrogène, alors t=0 et si p=0, alors t=1.

**6.** Composition selon l'une quelconque des revendication précédentes, **caractérisée en ce que** la ou les silicones non aminées sont choisies parmi les composés de formule (IIIa) suivante :

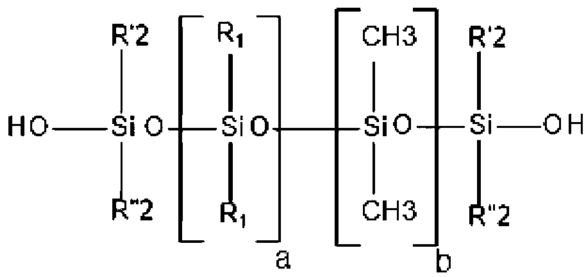

(IIIa)

dans laquelle :

- $\mathbf{R_1}$ représente indépendamment un groupement hydroxy ; un groupe alkyl ayant de 1 à 10 atomes de carbone, de préférence un méthyl ; un groupe aryl ayant de 6 à 12 atomes de carbone ; un groupe alcoxy ayant de 1 à 2 atomes de carbone ; ou un groupe -O-Si(R'$_2$)$_3$ ;
- $\mathbf{R'_2}$ et $\mathbf{R''_2}$ représentent indépendamment un atome d'hydrogène ; un groupement hydroxy ; ou un groupe alkyl ayant de 1 à 10 atomes de carbone, de préférence un méthyl ;
- **a** désigne un nombre entier allant de 0 à 10, **b** désigne un nombre entier allant de 0 à 500, avec a+b≥4.

**7.** Composition selon la revendication 6, **caractérisée en ce que** la ou les silicones non aminées de formule (IIIa) sont telles que :

- $\mathbf{R_1}$ représente indépendamment un groupe alkyl ayant de 1 à 10 atomes de carbone, de préférence un méthyl ;
- $\mathbf{R'_2}$ et $\mathbf{R''_2}$ représentent indépendamment un groupement hydroxy ou un groupe alkyl ayant de 1 à 10 atomes de carbone, de préférence un groupe alkyl, plus préférentiellement un méthyl ;
- **b** désigne un nombre entier allant de 0 à 10, **a** désigne un nombre entier allant de 0 à 5, avec a+b≥4.

**8.** Composition selon l'une quelconque des revendication précédentes, **caractérisée en ce que** la ou les silicones non aminées de formule (III) sont présentes en une quantité totale allant de 0,1% à 30% en poids, de préférence de 0,1% à 25% en poids par rapport au poids total de la composition.

**9.** Composition selon l'une quelconque des revendication précédentes, **caractérisée en ce que** le ratio massique d'alcoxysilane/silicone non aminée varie de 90 :10 à 10 :90, préférentiellement de 60 :40 à 40 :60, encore plus préférentiellement le ratio massique d'alcoxysilane/silicone non aminée est de 50 :50.

**10.** Composition selon l'une quelconque des revendications précédentes, **caractérisée en ce que** la composition (C) comprend un ou plusieurs solvants organiques choisis parmi les alcanols inférieurs en $C_1$-$C_4$, plus préférentiellement l'éthanol.

**11.** Utilisation de la composition telle que définie selon l'une quelconque des revendications 1 à 10, pour le traitement cosmétique, en particulier la coloration, des fibres kératiniques, en particulier des fibres kératiniques humaines telles que les cheveux.

**12.** Dispositif de traitement des fibres kératiniques humaines telles que les cheveux, comprenant deux compartiments contenant :

- dans un premier compartiment (C1), une composition (A) comprenant au moins un alcoxysilane choisi parmi les composés de formule (I), leurs oligomères et/ou leurs mélanges tel que défini selon l'une quelconque des revendications 1 à 4;
- dans un deuxième compartiment (C2), une composition (B) comprenant au moins une silicone non aminée choisie parmi les composés de formule (III) telle que définie selon l'une quelconque des revendications 1, et 5 à 8; et au moins un agent colorant choisi parmi les pigments, les colorants directs et leurs mélanges;

étant entendu que la composition (A) et/ou la composition (B) comprennent de l'eau et le mélange des compositions des compartiments (C1) et (C2) conduit à un ratio massique d' alcoxysilane/silicone non aminée variant de 95 :5 à 5 :95.

**13.** Procédé de traitement des fibres kératiniques humaines telles que les cheveux consistant à mélanger extemporanément au moment de l'emploi deux compositions (A) et (B) et à appliquer le mélange sur les fibres, le dit mélange comprenant un ratio massique d' alcoxysilane/silicone non aminée variant de 95 :5 à 5 :95, avec :

- la composition (A) comprenant au moins un alcoxysilane choisi parmi les composés de formule (I), leurs oligomères et/ou leurs mélanges tel que défini selon l'une quelconque des revendications 1 à 4;
- la composition (B) comprenant au moins une silicone non aminée choisie parmi les composés de formule (III) telle que définie selon l'une quelconque des revendications 1, et 5 à 8; et au moins un agent colorant choisi parmi les pigments, les colorants directs et leurs mélanges ;

étant entendu que la composition (A) et/ou la composition (B) comprennent de l'eau.

**14.** Procédé selon la revendication 13, **caractérisé en ce que** le ratio massique d'alcoxysilane/silicone non aminée varie de 90 :10 à 10 :90, de préférence de 60 :40 à 40 :60, plus préférentiellement le ratio massique d'alcoxysilane/silicone non aminée est de 50 :50.

**15.** Procédé de coloration des fibres kératiniques, en particulier humaines de préférence les cheveux, comprenant :

- l'application sur lesdites fibres d'une composition comprenant:

  a) au moins un alcoxysilane choisi parmi les composés de formule (I), leurs oligomères et/ou leurs mélanges tel que défini selon l'une quelconque des revendications 1 à 4,
  b) au moins une silicone non aminée choisie parmi les composés de formule (III) telle que définie selon l'une quelconque des revendications 1, et 5 à 8;
  c) de l'eau et
  d) au moins un agent colorant choisi parmi les pigments, les colorants directs et leurs mélanges.

**16.** Procédé de coloration selon la revendication 15, **caractérisé en ce que** le ratio massique d'alcoxysilane/silicone

non aminée varie de de 95 :5 à 5 :95, de préférence de 90 :10 à 10 :90, plus préférentiellement de 60 :40 à 40 :60, encore plus préférentiellement le ratio massique d'alcoxysilane/silicone non aminée est de 50 :50.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 2936414 A1
- FR 2679771
- EP 1184426 A
- JP 09188830 A
- JP 10158450 A
- JP 10158541 A
- JP 07258460 A
- JP 05017710 A
- US 4578266 A
- WO 9515144 A
- WO 9501772 A
- EP 714954 A


### Non-patent literature cited in the description


- *Cosmetics and Toiletries,* February 1990, vol. 105, 53-64